# EUROPEAN PATENT APPLICATION

(11) **EP 4 282 855 A1**
(43) Date of publication of application: **29.11.2023**
(21) Application number: 21924210.4
(22) Date of filing: 22.09.2021
(51) Int. Cl.: C07C 229/12, C07C 227/18, A61K 9/51, A61K 47/18, A61K 31/7088

(54) **IONIZABLE LIPID MOLECULE, PREPARATION METHOD THEREFOR, AND APPLICATION THEREOF IN PREPARATION OF LIPID NANOPARTICLE**

(30) Priority: 05.02.2021 CN 202110159969
(71) Applicant: IMMORNA (HANGZHOU) BIOTECHNOLOGY CO., LTD., Hangzhou City, Zhejiang Province 311217 (CN)
(72) Inventor: WANG, Zihao, Hangzhou City, Zhejiang 311231 (CN)
(74) Representative: Manitz Finsterwald Patent- und Rechtsanwaltspartnerschaft mbB
(86) International application number: PCT/CN2021/119577
(87) International publication number: WO 2022/166213

(57) **Abstract**

Disclosed in the present invention are an ionizable lipid molecule, a preparation method therefor, a composition containing same, an application thereof in the preparation of a vector for delivering a nucleic acid to a cell, and an application thereof in the preparation of a lipid nanoparticle (LNP). By improving the structure of the ionizable lipid molecule and adjusting an LNP component solution, the effect of an mRNA-LNP preparation is improved. The ionizable lipid molecule of the present invention has a structure of formula (I). The ionizable lipid molecule is synthesized with phospholipid, cholesterol, and polyethylene glycol by means of microfluidics to obtain the LNP, and the obtained LNP can improve the translation expression level of a load-mRNA in the cell, improve the effect of the mRNA-LNP preparation, and provide a theoretical basis for theoretical treatment of the personalized mRNA-LNP preparation.

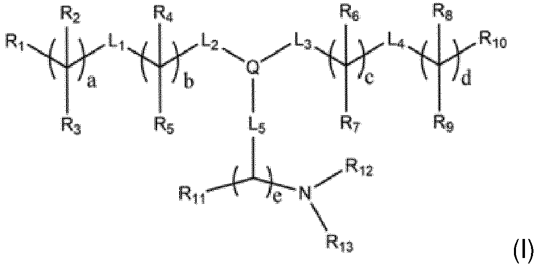

## Description

### Priority of Reference

The present application claims priority to CN202110159969.3, which is incorporated herein by reference in its entirety.

### FIELD OF THE INVENTION

The invention relates to the technical field of biology, in particular, to an ionizable lipid molecule, a preparation method therefor and use thereof in preparation of lipid nanoparticles.

### BACKGROUND OF THE INVENTION

Therapeutic nucleic acids include small interfering RNA (siRNA), microRNA (miRNA), and messenger RNA (mRNA). Such nucleic acids act through a variety of mechanisms. In the case of siRNA or miRNA, intracellular levels of specific proteins can be down-regulated by RNA interference (RNAi) method. After being introduced into the cytoplasm, siRNA or miRNA can bind to a RISC protein, its sense chain is removed by the RISC complex, thus providing a template within the RISC that can recognize and bind to mRNA having a complementary sequence to the bound siRNA or miRNA sequence. Upon binding to the complementary mRNA, the RISC complex cleaves the mRNA and releases the cleaved chain. RNAi can provide downregulation of specific proteins through targeted specific destruction of the corresponding mRNA encoding protein synthesis.

The therapeutic application of the RNAi technique is extremely wide because any nucleotide sequence for a target protein can be used to construct a corresponding siRNA or miRNA. Currently, siRNA or miRNA constructs have shown the ability to specifically down-regulate target proteins in in-vitro and in-vivo models. However, the use of siRNA or miRNA as systemic therapeutic agents will face two problems. First, they are very sensitive to nucleases in plasma; second, when siRNA or miRNA is systemically administered, its ability to enter intracellular compartments where it can bind to RISC is limited. To stabilize such double-stranded molecules, chemically modified nucleotide linkers, such as thiophosphate groups, may be incorporated into the molecule. However, this chemical modification provides only limited protection and may reduce the activity of the molecule. Other methods include using a carrier delivery system such as polymers, ionizable lipid molecules, or chemically modifying such double-stranded molecules (e.g., by covalent linkage of ionizable lipid molecules) to facilitate intracellular delivery of siRNA or miRNA.

mRNAs control the synthesis of proteins needed by living cells by transferring instructions stored in DNAs. The mRNA therapy involves allowing chemically modified mRNA molecules to enter the cytoplasm and utilize original nucleotides in the cytoplasm for transcriptional expression to generate proteins needed by organisms. For mRNA vaccines, the mRNA is modified *in vitro* correspondingly and then transferred to the body for expression in cells to produce protein antigen, thus inducing the body to produce immune response against the antigen to expand the body's immunity. Theoretically, mRNA can express any protein and thus can be used to treat various diseases and produce various vaccines.

mRNAs can be divided into two types: non-replicating mRNAs and self - amplifying mRNAs: the non-replicating mRNA only encodes the target protein and contains 5' and 3' untranslated regions (UTRs); and the self-amplifying mRNA encodes not only the target protein but also the replication mechanism that enables intracellular RNA amplification and protein expression. Self-amplifying mRNA allows for sustained high expression of the target protein compared to non-replicating mRNA, i.e. it can be administrated at lower doses while achieving the same therapeutic or immune effect.

One of the challenges of mRNA-based therapy is short half-life in that mRNAs can be rapidly degraded by a large number of extracellular RNA enzymes. In addition, mRNAs are immunogenic, that is, an exogenous RNA is generally considered by the body as a signal of viral infection. The spatial structure of mRNA can be stabilized, and its immunogenicity reduced to achieve high expression level of protein through various chemical modifications to the mRNA structure, such as modification of the 5'-cap, the polyadenylic acid (A) tail, the 5'- and 3'-UTR, and coding regions, replacement of natural adenosine with N1-methyladenosine (m1A) or N6-methyladenosine (m6A). In addition, most cells take in mRNAs inefficiently because the size of mRNA is significantly larger than that of an siRNA or miRNA construct. To date, researchers have used carrier delivery systems based on various lipid materials, such as various vesicles, liposomes, lipid nanoparticles (LNPs), lipid emulsions, and lipid implants to deliver chemically modified or unmodified mRNAs into cells.

Nucleic acids (RNAs) have great therapeutic potential in the regulation of protein expression *in vivo,* but the cellular internalization efficiency for naked RNA is extremely low, and during *in vivo* delivery, it is susceptible to quick clearance by the kidney, or quick degradation by RNA enzymes *in vivo,* which greatly discounts the actual efficacy. In order to more safely and effectively exert the therapeutic capability of RNAs, scientists use lipid nanoparticles (LNPs) to package and deliver RNAs to specific sites of the body. This RNA delivery strategy shows great application value in the delivery of double-stranded small interfering RNAs (siRNAs, 21 to 23 nucleotides long). For example, the lipid-like C12-200 has been widely used in the manufacture of siRNA-LNP preparations for a variety of therapeutic applications *in vivo* to inhibit protein expression. The emergence of synthetic ionizable lipids and lipid-like materials not only greatly reduces the *in vivo* toxicity of LNPs, but also makes it possible to deliver high molecular weight RNAs (such as mRNAs) *in vivo.* These amine-containing ionizable lipids or lipid-like molecules have a positive charge and can efficiently combine with negatively charged mRNAs by electrostatic attraction and self-assemble to form LNPs. LNPs can increase the blood circulation time of RNAs and increase the uptake rate into cells. In cells, RNA is released into the cytoplasm through the endosome escape pathway, making specific proteins expressed, thereby exerting certain therapeutic effects.

There are mainly four raw material components for LNPs: 1) ionizable lipid or lipid-like molecule (such as DLin-KC2-DMA, DLin-MC3-DMA, and L319), which is a core component for achieving *in vivo* delivery of mRNA; 2) phospholipid molecule, which is a phospholipid that provides structure for the LNP bilayer and may also help the endosome to escape; 3) cholesterol, which enhances LNP stability, and promotes membrane fusion; and 4) polyethylene glycol such as DMG-PEG2000, which can control and reduce the particle size of the LNP and "protect" the LNP from non-specific endocytosis by immune cells.

*In vivo*, changes in the raw materials and composition of the LNP have profound effects on the physicochemical stability and mRNA efficacy of the mRNA - LNP preparation. The existing LNP component formula cannot fully exert the efficacy of the mRNA-LNP preparation and it needs to continuously adjust the structure of ionizable lipid molecules and carry out design optimization aiming at specific RNAs. The invention solves such problems.

### SUMMARY OF THE INVENTION

In order to solve the disadvantages of the prior art, an objective of the invention is to provide an ionizable lipid molecule, a preparation method thereof, a composition comprising the same, use thereof in preparation of a carrier for delivering a nucleic acid to a cell, and use thereof in preparation of a lipid nanoparticle (LNP), to enhance the efficacy of the mRNA-LNP preparation by improving the structure of the ionizable lipid molecule and adjusting the component formula of the LNP.

In order to achieve the above objective, the invention adopts the following technical solutions:
The invention provides an ionizable lipid molecule, a pharmaceutically acceptable salt thereof and a stereoisomer thereof, where the ionizable lipid molecule has a structure shown in formula (I):

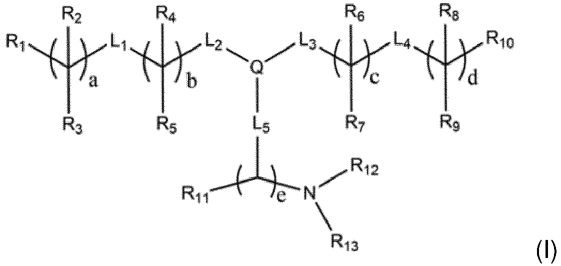

where,
Q is selected from a benzene ring, cyclopentyl, cyclohexyl, a pyrrole ring or a pyrimidine ring;
L₁ and L₄ are each independently selected from -O(C=O)-, -(C=O)O- or a carbon-carbon double bond;
L₂ and L₃ are each independently selected from -(CH₂)ₓO- or -O(CH₂)ₓ-, where x is an integer from 0 to 4;
L₅ is selected from -(CH₂)_{y}-O(C=O)-, -(CH₂)_{y}-(C=O)O- or -(CH₂)_{y}-O-, where y is an integer from 0 to 4;
L₂, L₃ and L₅ are each independently connected to any three sites in Q, where the sites are carbon or nitrogen;
a and d are each independently an integer from 0 to 18;
b and c are each independently an integer from 1 to 18;
e is an integer from 1 to 5;
R₁ and R₁₀ are each independently selected from methyl or cyclohydrocarbyl;
R₂ and R₃ are each independently selected from H or C₁-C₁₂ hydrocarbyl; or R₂ is selected from H or C₁-C₁₂ hydrocarbyl, and two carbon atoms connected to two adjacent R₃ form a carbon-carbon double bond;
R₄ and R₅ are each independently selected from H or C₁-C₁₂ hydrocarbyl; or R₄ is selected from H or C₁-C₁₂ hydrocarbyl, and two carbon atoms connected to two adjacent R₅ form a carbon-carbon double bond;
R₆ and R₇ are each independently selected from H or C₁-C₁₂ hydrocarbyl; or R₆ is selected from H or C₁-C₁₂ hydrocarbyl, and two carbon atoms connected to two adjacent R₇ form a carbon-carbon double bond;
R₈ and R₉ are each independently selected from H or C₁-C₁₂ hydrocarbyl; or R₈ is selected from H or C₁-C₁₂ hydrocarbyl, and two carbon atoms connected to two adjacent R₉ form a carbon-carbon double bond;
R₁₁ is independently selected from H or C₁-C₆ hydrocarbyl; and
R₁₂ and R₁₃ are each independently C₁-C₆ hydrocarbyl.

Preferably, where Q is selected from a benzene ring, cyclopentyl, or cyclohexyl; and more preferably, Q is a benzene ring.

Preferably, where L₂, L₃, and L₅ are connected to Q in a form of 1,2,5-trisubstitution, 1 ,3,5-trisubstitution, 1,2,4-trisubstitution, or 1,4,5-trisubstitution or 1,3,6-trisubstitution.

Preferably, where y is 0, 1, or 2.

Preferably, where a and d are each independently an integer from 6 to 10.

Preferably, where b and c are each independently an integer from 1 to 10.

Preferably, where e is selected from 2 or 3.

Preferably, where the cyclohydrocarbyl in R₁ and R₁₀ is selected from cyclopropyl, cyclobutyl, cyclopentyl, cyclohexyl or cycloheptyl;
the C₁-C₁₂ hydrocarbyl in the definitions of R₂ to R₉ is selected from C₁-C₁₂ alkyl, C₁-C₁₂ alkenyl, or C₁-C₁₂ alkynyl; and more preferably, the C₁-C₁₂ hydrocarbyl is C₁-C₆ alkyl;
R₁₁ is selected from H, methyl, ethyl, propyl, butyl, pentyl or hexyl; and more preferably is methyl; and
R₁₂ and R₁₃ are each independently selected from methyl, ethyl, propyl, butyl, pentyl or hexyl; and more preferably are methyl. By way of example, representative compounds I to XXV in the present application are given in Table 1.

**Table 1: Representative compounds of the present application**

| No. | Structure |
|---|---|
| I | 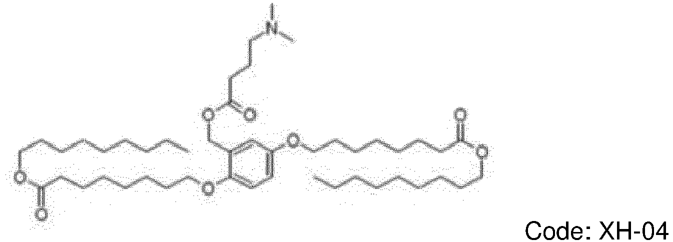 |
| II | 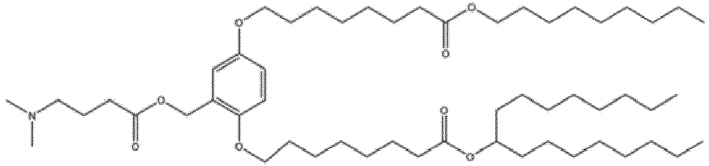 |
| III | 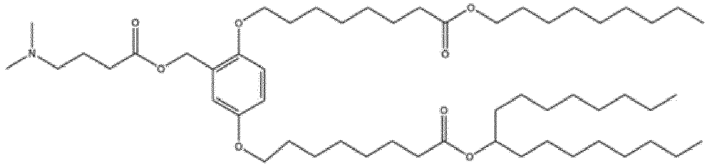 |
| IV | 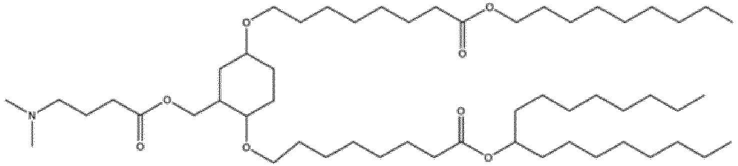 |
| V | 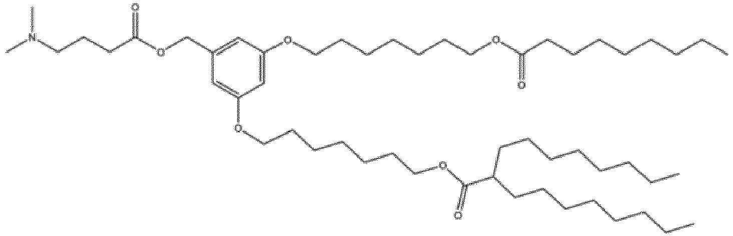 |
| VI | 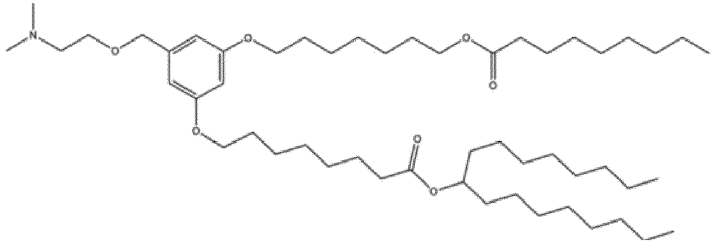 |
| VII | 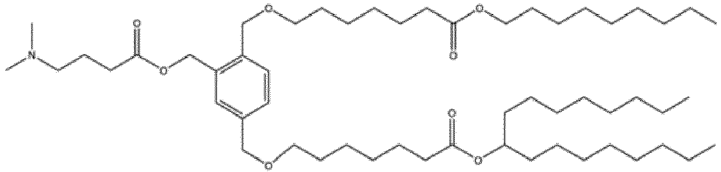 |
| VIII | 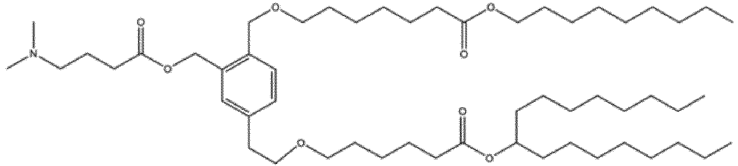 |
| IX | 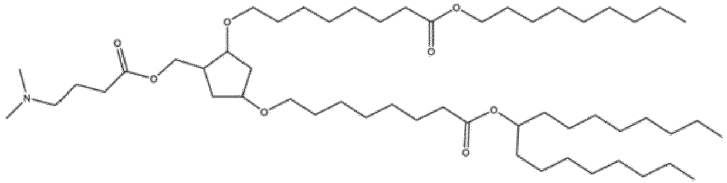 |
| X | 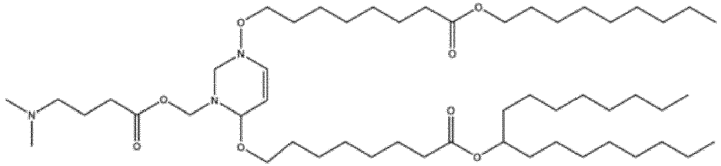 |
| XI | 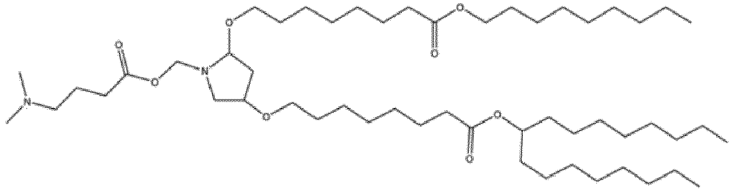 |
| XII | 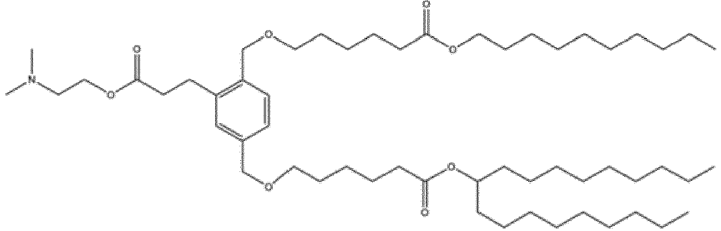 |
| XIII | 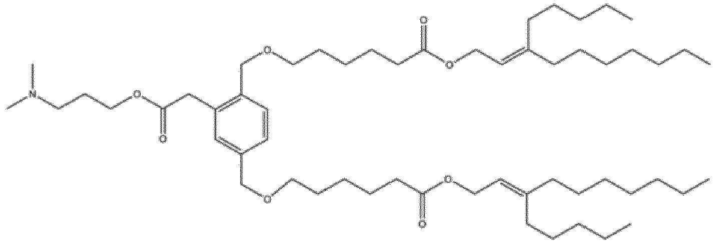 |
| XIV | 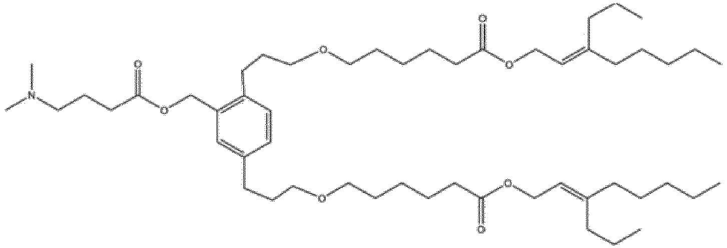 |
| XV | 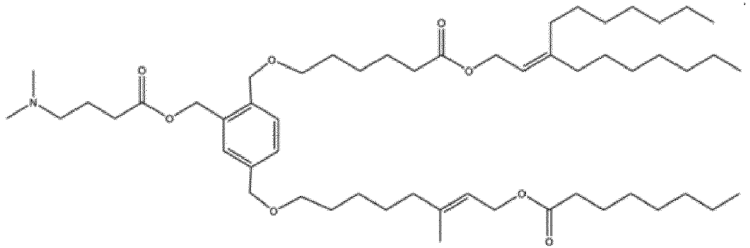 |
| XVI | 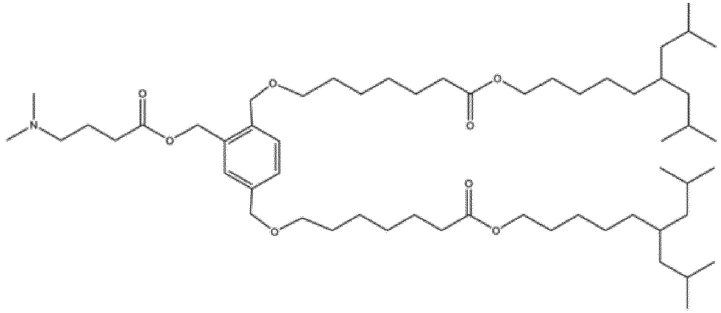 |
| XVII | 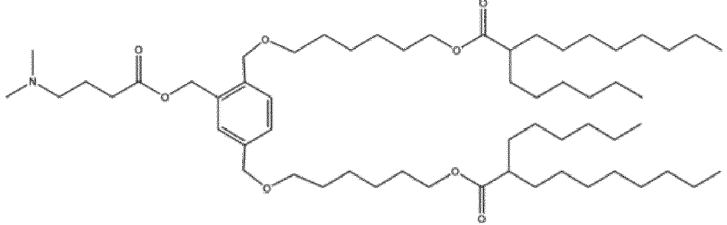 |
| XVIII | 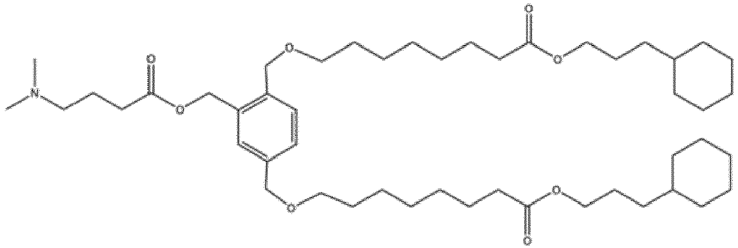 |
| XIX | 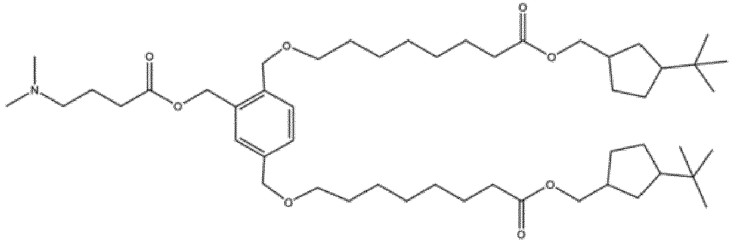 |
| XX | 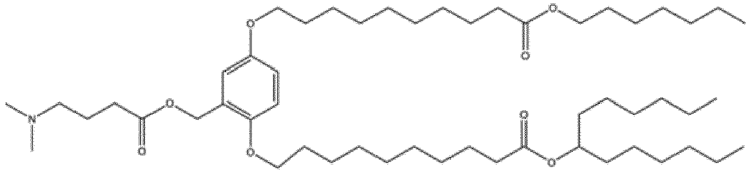 |
| XXI | 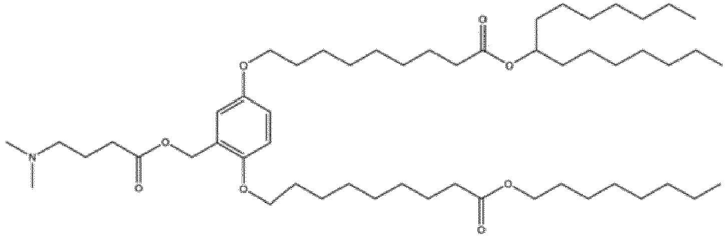 |
| XXII | 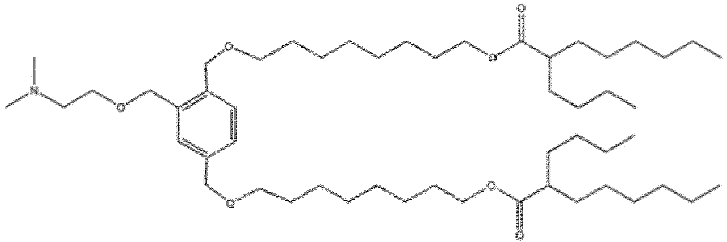 |
| XXIII | 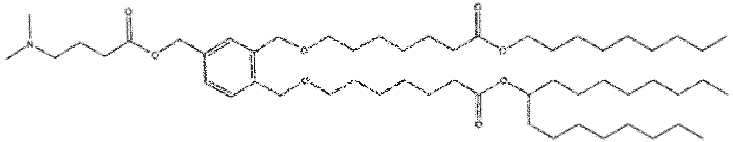 |
| XXIV | 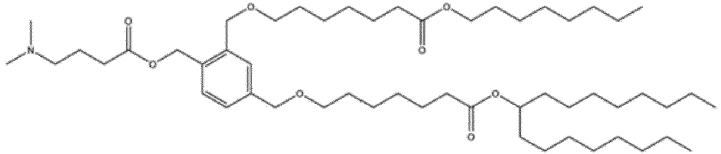 |
| XXV | 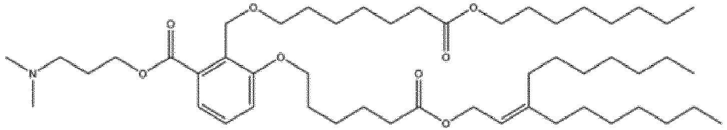 |

Preferably, the ionizable lipid molecule is compound I:

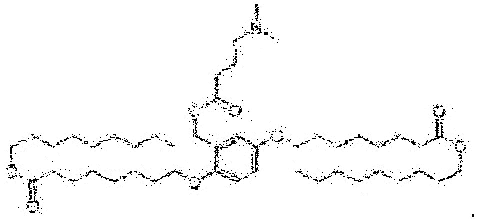

Preferably, the ionizable lipid molecule is compound II:

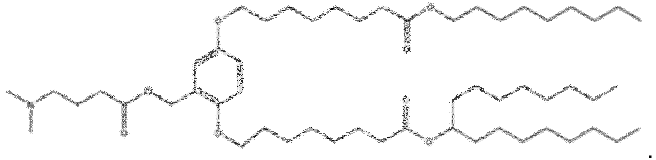

Preferably, the ionizable lipid molecule is compound III:

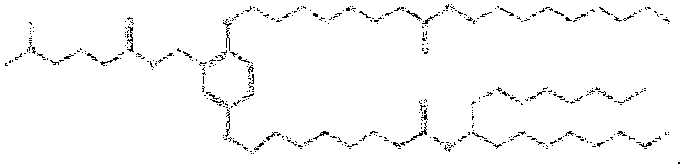

The invention provides a preparation method of the ionizable lipid molecule, which, taking compound I as an example, comprises the following steps:
step 1: to a mixed solution comprising 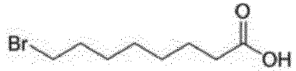 with dichloromethane (DCM) as a solvent, 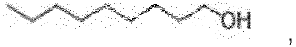 4-dimethylaminopyridine (DMAP) and triethylamine (TEA) are added; then 1-ethyl-(3-dimethylaminopropyl)carbodiimide hydrochloride (EDCI) is added to the solution with stirring for reaction; after completion of the reaction indicated by detection, the reaction solution is extracted, washed, dried, concentrated and purified to obtain 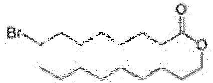 as a yellow oil;
step 2: to a mixed solution comprising 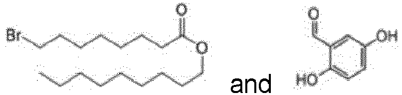 with N,N-dimethylformamide (DMF) as a solvent, Cs₂CO₃ is added, the mixture is stirred to react, and after completion of the reaction indicated by detection, the reaction solution is extracted, washed, dried, concentrated and purified to obtain 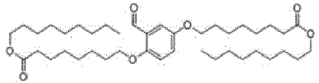 as a white solid;
step 3: to a mixed solution of tetrahydrofuran (THF), ethanol and 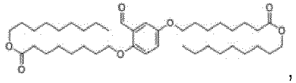 NaBH₄ below 0°C is added, the mixture is stirred to react, and after completion of the reaction indicated by detection, the reaction solution is extracted, washed, dried, and concentrated to obtain 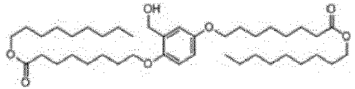 as a white solid; and
step 4: 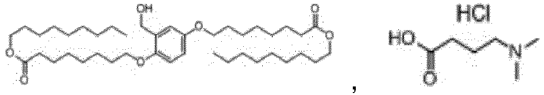 and 1-ethyl-(3-dimethylaminopropyl)carbodiimide hydrochloride (EDCI) are stirred to react in a pyridine solution, and after completion of the reaction indicated by detection, the reaction solution is extracted, washed, dried, concentrated and purified to obtain an ionizable lipid molecule 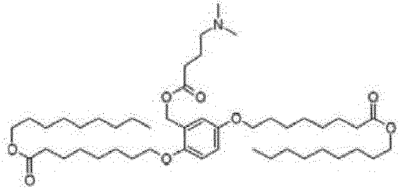 as a colorless oil.

In the aforementioned preparation method of the ionizable lipid molecule, the method for detecting completion of the reaction is silica gel plate thin layer chromatography (TLC).

The invention provides a composition comprising the ionizable lipid molecule, pharmaceutically acceptable salt thereof and stereoisomer thereof, and a therapeutic agent.

Preferably, where the therapeutic agent includes a nucleic acid.

Preferably, where the nucleic acid is selected from small interfering RNA (siRNA), microRNA (miRNA), and messenger RNA (mRNA).

Preferably, where the mRNA is non-self-replicating mRNA or self-replicating mRNA.

Preferably, where the composition comprises one or more excipients selected from a neutral lipid, a steroid, and a polymer-conjugated lipid;
preferably, the neutral lipid is selected from one or more of 1,2-distearoyl-sn-glycerophosphatidylcholine (DSPC), dipalmitoyl phosphatidylcholine (DPPC), dimyristoyl phosphatidylcholine (DMPC), dioleoyl phosphatidylcholine (DOPC), palmitoyl oleoyl phosphatidylcholine (POPC), and 1,2-dioleoyl-sn-glycero-3-phosphoethanolamine (DOPE); and more preferably DSPC and DOPE;
the steroid is selected from cholesterol, sitosterol, fucosterol, campesterol, and stigmasterol; and more preferably is cholesterol; and
the polymer-conjugated lipid is polyethylene glycol, which is preferably PEGylated DMG, and more preferably DMG-PEG2000, where DMG represents 1,2-dimyristoyl-sn-glycerol.

Preferably, where the molar ratio of the ionizable lipid molecule to the neutral lipid is 2:1 to 8:1 and the molar ratio of the ionizable lipid molecule to the cholesterol is 2:1 to 1:1; and the molar ratio of the ionizable lipid molecule to the PEGylated DMG is 100:1 to 25:1.

The invention also provides use of the ionizable lipid molecule, pharmaceutically acceptable salt thereof and stereoisomer thereof in preparation of a carrier for delivering a nucleic acid to a cell.

Preferably, the nucleic acid is selected from small interfering RNA (siRNA), microRNA (miRNA), and messenger RNA (mRNA).

More preferably, where the mRNA is non-self-replicating mRNA or self-replicating mRNA.

The invention provides use of the ionizable lipid molecule, pharmaceutically acceptable salt thereof and stereoisomer thereof in preparation of a lipid nanoparticle, which comprises obtaining a liposome nanoparticle (LNP) by synthesis from the ionizable lipid molecule, as well as phospholipid, cholesterol, and polyethylene glycol, the LNP being capable of improving the translational expression level of its cargo, i.e. mRNA, in a cell.

Preferably, where the polyethylene glycol is preferably PEGylated DMG, and more preferably DMG-PEG2000.

Preferably, where the ionizable lipid molecule is selected from compounds I to III:
compound I: 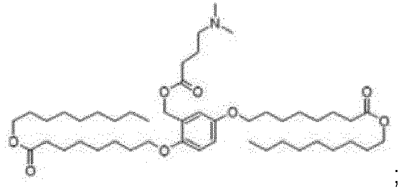
compound II: 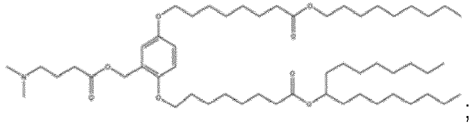 and
compound III:

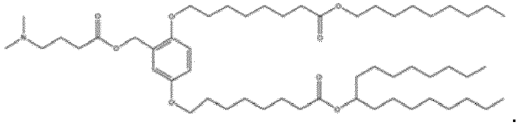

Preferably, where the phospholipid is DSPC or DOPE.

Preferably, where the formula for synthesis of the carrier LNP is based on the following molar percentages: ionizable lipid molecule (e.g., any one of compounds I to III): cholesterol: phospholipid: polyethylene glycol = 20-70%: 20-60%: 2-20%: 0.1-5%.

More preferably, where when compound I is used, the formula for synthesis of the carrier LNP is based on the following molar percentages: ionizable lipid molecule: cholesterol: DSPC: DMG-PEG2000 = 31.5%: 56%: 10%: 2.5%. When compound II or compound III is used, the formula for synthesis of the carrier LNP is based on the following molar percentages: ionizable lipid molecule: cholesterol: DSPC: DMG-PEG2000 = 38.5%: 50%: 10%: 1.5%.

Preferably, where the LNP is obtained by a microfluidic synthesis method using the ionizable lipid molecule, phospholipid, cholesterol, and polyethylene glycol.

Preferably, the microfluidic synthesis method comprises steps of:
preparing a mixed solution of the ionizable lipid molecule and phospholipid, cholesterol and polyethylene glycol;
preparing an mRNA diluent;
preparing the LNP using a microfluidic synthesizer with microfluidic chips provided by the manufacturer: the mRNA diluent is loaded into a syringe on one side, the mixed solution is loaded into a springe on the other side, the parameters are set, the synthesis procedure is repeated several times to use up all the mixed solution, all the product LNP solutions are collected, and a citric acid buffer is added within 30 min to dilute the solution to have less than 0.5% of ethanol proportion in the solution.

Preferably, the microfluidic synthesis method further comprises purification by tangential flow filtration to obtain the LNP.

The invention provides preparation of a lipid nanoparticle composition comprising enhanced green fluorescent protein mRNA (eGFP_mNRA) by dissolving ionizable lipid, cholesterol, DSPC and DMG - PEG2000 at a molar ratio of 38.5:50:10:1.5 in ethanol. Lipid nanoparticles (LNPs) are prepared with a weight ratio of total lipids to mRNA of 10:1 to 30:1 (preferably, 10:1). Briefly, mRNA is diluted to 0.2 mg/mL in 10-50 mM (preferably, 50 mM) citrate buffer (pH 6). Using a syringe pump, the ethanol solution of the lipids is mixed with the aqueous solution of mRNA at a ratio of about 1:5 to 1:2 (volume/volume) (preferably, 1:4) with a total flow rate of more than 15 mL/min. The ethanol is then removed, and the external buffer is replaced by PBS through dialysis. Finally, the lipid nanoparticles are filtered through a 0.22 µm pore sterile filter. The particle size of the lipid nanoparticles determined by quasi-elastic light scattering is 80-100 nm in diameter.

The invention is based on the discovery of novel ionizable lipid molecules that provide advantages when used in lipid nanoparticles for *in vivo* delivery of an active agent or therapeutic agent (e.g., nucleic acid) into mammalian cells. In particular, embodiments of the invention provide nucleic acid-lipid nanoparticle compositions comprising one or more of the novel ionizable lipid molecules described herein, which provide enhanced nucleic acid activity and enhanced tolerance of the composition in the body, enabling a significant increase in therapeutic effect index as compared to previously described nucleic acid-lipid nanoparticle compositions.

The above formula (I) may be used alone or in combination with other lipid components such as neutral lipids, charged lipids, steroids (including, for example, all sterols) and/or their analogues and/or polymer-conjugated lipids to form lipid nanoparticles for delivery of therapeutic agents. In some examples, the lipid nanoparticles are used to deliver nucleic acids, such as siRNA, miRNA, and/or mRNA. Methods of using such lipid nanoparticles for the treatment of various diseases or conditions, such as those caused by solid tumors and/or protein deficiency, are also provided. Also provided is a method of administering a therapeutic agent to a patient in need thereof, the method comprising preparing a composition comprising lipid nanoparticles of the compound of formula (I) and a therapeutic agent, and delivering the composition to the patient.

In particular embodiments, the invention provides novel ionizable lipids capable of forming improved compositions for *in vitro* and *in vivo* delivery of mRNAs and/or other oligonucleotides. In some embodiments, these improved lipid nanoparticle compositions are useful for the expression of proteins encoded by mRNAs. In some other embodiments, the lipid nanoparticles are also useful for delivery of mRNAs and plasmids for transgene expression. In other embodiments, the lipid nanoparticle composition is useful for eliciting a pharmacological effect resulting from protein expression, for example, by delivering a suitable erythropoietin mRNA to produce increased red blood cells, or by delivering an mRNA for encoding a suitable antibody to prevent infection.

The pharmaceutical compositions of the invention may be formulated into the formulation in solid, semi-solid or liquid forms such as ointments, solutions, suspensions, injections, inhalants, gels, microspheres and aerosols. Typical routes of administration of such pharmaceutical compositions include, but are not limited to, oral, topical, transdermal, inhalation and intranasal routes, including subcutaneous injection, intravenous, intramuscular, intradermal or infusion techniques.

The liquid pharmaceutical compositions of the invention, whether in solution, suspension or other similar forms, may include one or more of the following adjuvants: sterile diluents such as water for injection, saline solutions, preferably normal saline, Ringer's solution, isotonic sodium chloride; non-volatile oils such as synthetic monoglycerides or diglycerides, polyethylene glycol, glycerol, propylene glycol or other solvents useful as solvents or suspension media; antioxidants such as ascorbic acid or sodium bisulfite; chelating agents such as ethylenediaminetetraacetic acid; buffers such as acetate, trimethylolaminomethane, citrate or phosphate; and preparations for regulating tension, such as sodium chloride or glucose; and reagents for use as cryoprotectants, such as sucrose or trehalose. The preparations may be packaged in glass or plastic ampoules, disposable syringes or multi-dose vials. Normal saline is a preferred adjuvant. Injectable pharmaceutical compositions are preferably sterile.

When the pharmaceutical composition of the invention is used for topical administration, the carrier may suitably comprise a solution matrix, an emulsion matrix, an ointment matrix, or a gel matrix. For example, the matrix may comprise one or more of petrolatum, lanolin, polyethylene glycol, beeswax, mineral oil, diluents such as water and alcohols, and emulsifiers and stabilizers. A thickening agent may be present in a pharmaceutical composition for topical administration. If used for transdermal administration, the composition may comprise a transdermal patch or an iontophoretic device.

The composition of the invention may be administered simultaneously with, before or after the administration of one or more other therapeutic agents. Such combination therapies include administration of a single pharmaceutical dose formulation of the composition of the invention and one or more additional active agents, as well as administration of the composition of the invention and the active agents in their separate pharmaceutical dose formulations. For example, the composition of the invention and other active agents may be administered to a patient together in a single injectable dose composition (e. g., an injection), or the individual agents may be administered as separate injectable dose formulations. When different dose formulations are used, the compound of the invention and one or more additional active agents may be administered at substantially the same time or at staggered time points.

The benefits of the invention are as follows:
The invention provides a novel class of ionizable lipid molecules by improving the structure of ionizable lipid molecules that are capable of efficiently delivering nucleic acids into the cytoplasm of cells.

According to the invention, LNPs are obtained by synthesis using the ionizable lipid molecule, as well as phospholipid, cholesterol and polyethylene glycol, and the resulting LNPs have more excellent performance as an mRNA carrier, and can obviously improve the translational expression level of its cargo, i.e. mRNA, in cells.

### BRIEF DESCRIPTION OF DRAWINGS

Fig. 1 is a synthesis flow diagram of one example of the preparation method of the invention;
Fig. 2 is a cryo-EM image of XH-04LNP prepared according to the invention;
Fig. 3 is a flow cytometry chart showing the expression levels of spike protein in BHK cells for the MC3-LNP (a) and XH-04-LNP (b) of the invention; and
Fig. 4 is the mean intensity of fluorescent protein expressed in BHK cells for compositions of lipid nanoparticles prepared using different ionizable lipid molecules and enhanced green fluorescent protein mRNA at different concentrations.

### DETAILED DESCRIPTION OF THE INVENTION

The invention is described in detail below with reference to the accompanying drawings and specific Examples. The Examples are intended to be illustrative and should not be construed as limiting the scope of the claims of the present application.

### Examples

The following abbreviations represent the following reagents respectively:
DCM: dichloromethane; DMAP: 4-dimethylaminopyridine; TEA: trimethylamine; EDCI: 1-ethyl-(3-dimethylaminopropyl)carbodiimide hydrochloride; TLC: silica gel plate thin layer chromatography; THF: tetrahydrofuran; DMF: N, N-dimethylformamide; DSPC: 1,2-distearoyl-sn-glycerophosphatidylcholine; DOPE: 1,2-dioleoyl-sn-glycero-3-phosphoethanolamine; TFF: tangential flow filtration.

### Example 1 Synthesis of compound I (code: XH-04)

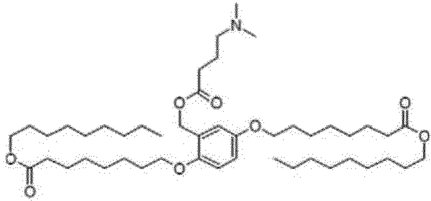

(compound I, code: XH-04)

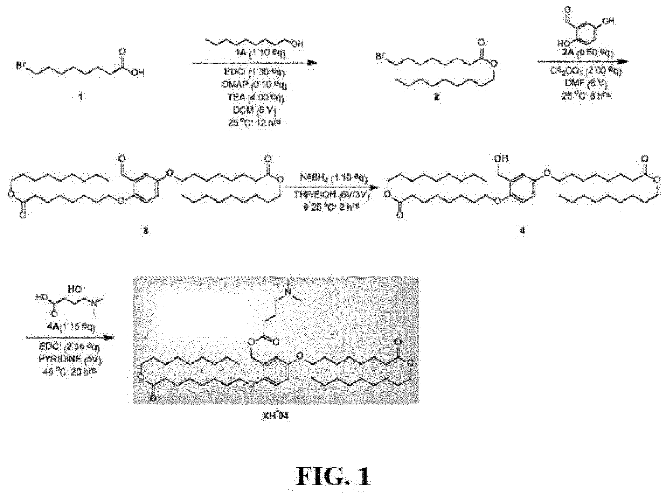

The preparation method of compound I is as shown in Fig. 1, specifically comprising the following steps:
Step 1 To a solution of compound 1 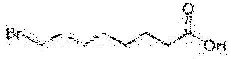 (20.0 g, 89.6 mmol, 1.00 eq.) in dichloromethane (DCM) (100 mL), compound 1A 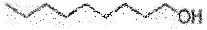 (14.2 g, 98.6 mmol, 1.10 eq.), 4-dimethylaminopyridine (DMAP) (1.10 g, 8.96 mmol, 0.10 eq.) and trimethylamine (TEA) (36.3 g, 358.6 mmol, 49.9 mL, 4.00 eq.) were added. To the solution was added 1 -ethyl-(3-dimethylaminopropyl)carbodiimide hydrochloride (EDCI) (22.3 g, 116.5 mmol, 1.30 eq.). The reaction was stirred at 25°C for 12 h. Silica gel plate thin layer chromatography (TLC) (petroleum ether: ethyl acetate=10:1, Rf= 0.62) showed the reaction was complete. The reaction solution was poured into H₂O (100 mL) and extracted with dichloromethane (200 mL × 2). The organic layer was rinsed with saline (100 mL), dried over sodium sulfate and concentrated under reduced pressure to give a crude product. The crude product was purified by column chromatography (SiO2, petroleum ether: ethyl acetate = 1:0 to 0:1) to obtain compound 2 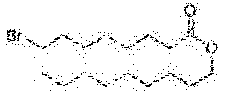 as a yellow oil (22.0 g, 63.0 mmol, 70.3%). ¹H NMR: (400MHz, CDCl₃) δ 4.03 (t, J=6.8Hz, 2H), 3.38 (t,J=6.0Hz, 2H), 2.27 (t,J=7.6Hz, 2H), 1.71-1.86 (m, 2H), 1.56-1.61 (m, 4H), 1.40-1.43 (m, 2H), 1.22-1.33 (m, 16H), 0.84-0.88 (m, 3H).

The reaction formula of step 1 is as follows:

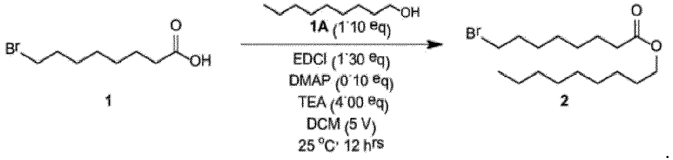

Step 2: To a solution of compound 2 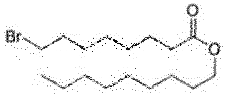 (20.0 g, 57.25 mmol, 1.00 eq.) and compound 2A 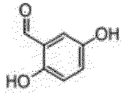 (3.95 g, 28.6 mmol, 0.50 eq.) in dimethylformamide (DMF) (120 mL) was added Cs₂CO₃ (37.3 g, 114.5 mmol, 2.00 eq.). The suspension was stirred at 25°C for 6 h. Silica gel plate thin layer chromatography (TLC) (petroleum ether: ethyl acetate = 8: 1, Rf=0.40) showed the reaction was complete. The reaction solution was poured into H₂O (200 mL) and extracted with dichloromethane (200 mL × 3). The organic layer was rinsed with saline (100 mL), dried over sodium sulfate and concentrated under reduced pressure to give a crude product. The crude product was purified by column chromatography (SiO₂, petroleum ether: ethyl acetate = 1:0 to 10:1) to obtain compound 3: dinonyl-8,8'-((2-formyl-1,4-phenylene)dioxy)octanoic acid as a white solid (15.0 g, 22.2 mmol, yield 77.6%). 1H NMR: (400MHz, CDCl3) δ 10.47 (s, 1H), 7.31 (d,J=3.2Hz, 1H), 7.10 (dd, J1 =8.8Hz, J2=3.2Hz, 1H), 6.92 (d,J=9.2Hz, 1H), 4.01 -4.08(m, 6H), 3.94(t,J=6.4Hz, 2H), 2.31 (t,J=7.2Hz, 4H), 1.74-1.84 (m, 4H), 1.59-1.68 (m, 8H), 1.27-1.43 (m, 36H), 0.87-0.90 (m, 6H).

The reaction formula of step 2 is as follows:

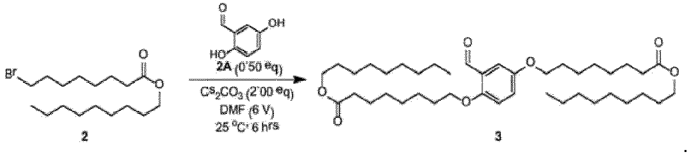

Step 3: To a solution of compound 3 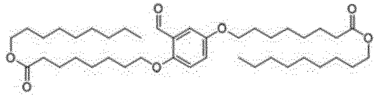 (12.0 g, 17.78 mmol, 1.00 eq.) in tetrahydrofuran (THF) (72 mL) and ethanol (EtOH) (36 mL) was added NaBH₄ (739.9 mg, 19.6 mmol, 1.00 eq.) at 0°C. After the addition, the reaction solution was stirred at 25°C for 2 h. Silica gel plate thin layer chromatography (TLC) (petroleum ether: ethyl acetate = 5: 1, Rf=0.20) showed the reaction was complete. The reaction solution was poured into H₂O (100mL) and extracted with dichloromethane (200 mL × 2). The organic layer was rinsed with saline (200 mL), dried over sodium sulfate and concentrated under reduced pressure to afford compound 4 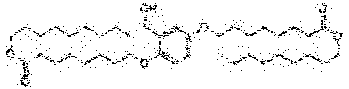 as a white solid (11.8 g, crude). ¹H NMR: (400MHz, CDCl₃) δ 6.87(d,J=2.4Hz, 1H), 6.71-6.76(m, 2H), 4.63(s, 2H), 4.04(t,J=6.8Hz, 4H), 3.93(t,J=6.4Hz, 2H), 3.88(t,J=6.4Hz, 2H), 3.69-3.74(m,1H), 2.28(t, J=7.2Hz, 4H), 1.69 -1.78(m, 4H), 1.56-1.65(m, 8H), 1.21-1.45(m, 36H), 0.84-0.88(m, 6H).

The reaction formula of step 3 is:

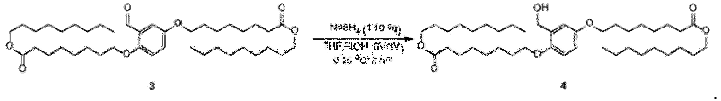

Step 4: A solution of compound 4 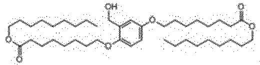 (11.0 g, 16.3 mmol, 1.00 eq.), compound 4A 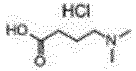 (3.13 g, 18.69 mmol, 1.15 eq., HCl) and 1-ethyl-(3-dimethylaminopropyl)carbodiimide hydrochloride (EDCI) (7.16 g, 37.4 mmol, 2.30 eq.) in pyridine (55 mL) was stirred at 40°C for 20 h. Silica gel plate thin layer chromatography (TLC) (dichloromethane: methanol = 10:1, Rf=0.39) showed the reaction was complete. The reaction solution was poured into to a saturated ammonium chloride solution (60 mL), and extracted with ethyl acetate (120 mL × 3). The organic layer was dried over sodium sulfate and concentrated under reduced pressure to give a crude product. The crude product was purified by silica gel column chromatography (petroleum ether: ethyl acetate = 100:1 to 0:1) to obtain compound I, XH-04, as a colorless oil (4.00 g, 5.06 mmol, yield 31.2%). ¹H NMR: (400MHz, CDCl₃) δ 6.89(s, 1H), 6.77(d,J=2.4Hz, 2H), 5.14(s, 2H), 4.06(t,J=6.8Hz, 4H), 3.90(q,J=6.4Hz, 4H), 2.30-2.42(m, 4H), 2.26-2.29(m, 10H), 1.82-1.89(m, 2H), 1.72-1.78(m, 4H), 1.27 -1.45(m, 36H), 0.86-0.90(m, 6H).

The reaction formula of step 4 is as follows:

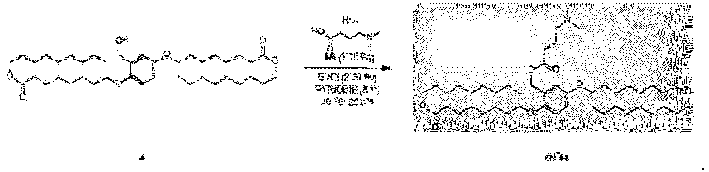

### Example 2 Synthesis of compound II

### Compound II:

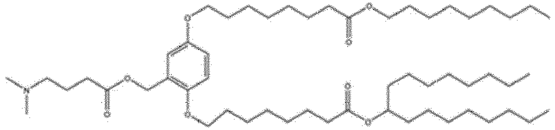

To a solution of nonanol (19.4 g) and bromooctanoic acid (30 g) in dichloromethane (210 mL) was added triethylamine (40.8 g), dimethylaminopyridine (1.64 g) and 1-ethyl-(3-dimethylaminopropyl)carbodiimide hydrochloride (25.7 g), the mixture was stirred at room temperature for 12 h, and then a solution of hydrochloric acid (20 mL) was added to terminate the reaction. The reaction mixture was extracted 3 times with ethyl acetate. The filtrate was washed with brine, dried over anhydrous magnesium sulfate, filtered to remove the solvent, and purified on a chromatographic column to obtain 14 g of ethyl nonyl-8-bromooctanoate.

Triethylamine (68 g), dimethylaminopyridine (2.74 g) and 1-ethyl-(3-dimethylaminopropyl)carbodiimide hydrochloride (94.5 g) were added to a solution of heptadecylamine-1,9,17-triol (63.2 g) and bromooctanoic acid (50 g) in dichloromethane (350 mL). The mixture was stirred at room temperature for 12 h, and then a hydrochloric acid solution was added to terminate the reaction. The reaction mixture was extracted 3 times with ethyl acetate. The filtrate was washed with brine, dried over anhydrous magnesium sulfate, filtered to remove the solvent, and purified by a chromatographic column to obtain a colorless oil. To a solution of 5-bromo-2-hydroxybenzaldehyde (25 g) in dimethylformamide (175 mL) was added the colorless oil (47.7 g) and potassium carbonate (34.3 g), the mixture was stirred at 80°C for 12 h, and then deionized water (300 mL) was added to terminate the reaction. The reaction mixture was extracted 3 times with ethyl acetate and the filtrate was washed with brine. The organic portion was dried over anhydrous magnesium sulfate, filtered to remove the solvent, and purified by a chromatographic column to obtain 57g of 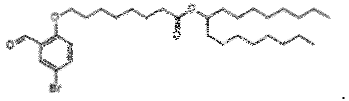 ¹H NMR: (400 MHz, CDCl₃) δ 10.41 (s, 1H), 7.92(d,J=2.4Hz, 1H), 7.61-7.59(m, 1H), 6.87(d,J=8.8Hz, 1H), 4.88-4.84(m, 1H), 4.05(t,J=6.4Hz, 2H), 2.31-2.27(m, 2H), 1.86-1.81(m, 2H), 1.71-1.64(m, 2H), 1.51-1.37(m, 36H), 0.87-0.85(m, 6H). 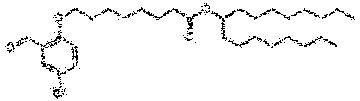 (20g) was dissolved in a mixed solution of tetrahydrofuran (40 mL) and methanol (100 mL), precooled to 0°C and treated with sodium borohydride (520 mg) for 3 h. An ammonium chloride solution (20 mL) was added to stop the reaction and the reaction mixture was extracted 3 times with ethyl acetate. The filtrate was washed with brine, dried over anhydrous magnesium sulfate, filtered, and purified on a chromatographic column to obtain a brown oil. The brown oil was dissolved in dimethylformamide (21 mL) and treated with p-toluenesulfonylimidazole (3.5 g) and t-butyl diphenyl chlorosilane (11.3 g) for 12 h and purified on a chromatographic column to obtain 24 g of a yellow oil.

10 g of the yellow oil was dissolved in dioxane (70 mL) and 3.71 g of 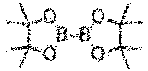 and [1,1'-bis(diphenylphosphino)ferrocene]palladium dichloride (890 mg) and potassium acetate (2.39 g) were added for treatment under nitrogen atmosphere for 12 h. The mixture was purified on a chromatographic column to obtain 13 g of a yellow oil. The yellow oil (13 g) was dissolved in tetrahydrofuran (130 mL) and treated by addition of hydrogen peroxide (3.39 g) and sodium hydroxide (1 M, 14.9 mL) for 1 h. Sodium thiosulfate was added to stop the reaction, and the reaction mixture was extracted with ethyl acetate for 2 time. The filtrate was washed with brine, dried over anhydrous magnesium sulfate, filtered, and purified on a chromatographic column to obtain a brown oily intermediate product 3, 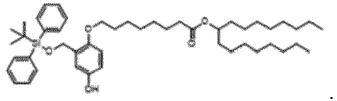 ¹H NMR: (400MHz, CDCl₃) δ 7.76-7.67 (m, 4H), 7.46-7.34 (m, 7H), 7.18 (s, 1H), 6.67 (s, 1H), 4.81 -4.72 (m, 1H), 4.69 (s,1H), 3.81 -3.78 (m, 2H), 2.25 (t,J=7.4Hz, 2H), 1.66-1.55 (m, 5H), 1.42-1.43 (m, 4H), 1.30-1.22 (m, 34H), 1.12 (s, 9H), 0.88 (t,J=6.8Hz, 6H).

A solution of the brown oily intermediate product 3 (9.5 g) in dimethylformamide (63 mL) was treated with ethyl nonyl-8-bromooctanoate (5.03 g) and potassium carbonate (3.46 g). The reaction mixture was stirred at 90°C for 12 h, and extracted 3 times with the solvent. The filtrate was washed with brine, dried over anhydrous magnesium sulfate, filtered and purified on a chromatographic column to obtain 6.3 g of a yellow oil. The yellow oil was dissolved in tetrahydrofuran (30 mL), and tetrabutyl ammonium fluoride (1 M, 12.2 mL) was added with stirring to react for 5 h, and then the reaction solution was extracted twice with ethyl acetate. The filtrate was washed with brine, dried over anhydrous magnesium sulfate, filtered and purified on a chromatographic column to obtain 6 g of a yellow oil. The yellow oil was dissolved in pyridine (6 mL), and 4-aminobutyric acid (1.91 g) and carbodiimide (2.91 g) were added. The mixture was stirred at 50°C to react for 12 h, concentrated under reduced pressure and purified on a chromatographic column to obtain 3 g of the end product, compound II (yield 43.7%). ¹H NMR: (400MHz, CDCl₃) δ 6.86 (s, 1H), 6.79 (s, 2H), 5.15 (s, 2H), 4.86 (t,J=6.2Hz, 1H), 4.06 (t,J=6.6Hz, 2H), 3.91 (td,J=6.4,9.0Hz, 4H), 3.09-2.91 (m, 2H), 2.74 (s, 6H), 2.52 (t,J=6.6Hz, 2H), 2.32-2.27 (m, 4H), 2.23-2.10 (m, 2H), 1.79-1.74 (m, 4H), 1.65-1.58 (m, 6H), 1.55-1.41 (m, 9H), 1.41-1.34 (m, 9H), 1.33-1.23 (m, 34H), 0.93-0.83 (m, 9H).

### Example 3: Synthesis of compound III

### Compound III:

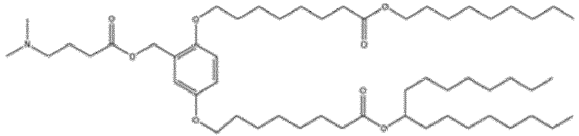

Compound III was synthesized with reference to the synthesis method of compound II. The H NMR of compound III is as follows:
¹H NMR: (400MHz, CDCl₃) δ 6.86 (s,1H), 6.79 (s, 2H), 5.15 (s, 2H), 4.86 (t,J=6.2Hz, 1H), 4.06 (t,J=6.6Hz, 2H), 3.91 (td,J=6.4,9.0Hz, 4H), 3.09-2.91 (m, 2H), 2.74 (s, 6H), 2.52 (t,J=6.6Hz, 2H), 2.32-2.27 (m, 4H), 2.23-2.10 (m, 2H), 1.79-1.74 (m, 4H), 1.65-1.58 (m, 6H), 1.55-1.41 (m, 9H), 1.41-1.34 (m, 9H), 1.33-1.23 (m, 34H), 0.93-0.83 (m, 9H).

### Example 4: Synthesis of compounds IV to XXV

Compounds IV to XXV were prepared with reference to the synthesis method of compound I and verified by nuclear magnetic resonance (NMR) and mass spectrometry (MS). Due to limited space in the specification, the synthesis steps and characterization data are not set forth in detail.

### Example 5 Use of compound I in preparation of lipid nanoparticles

Use of ionizable lipid molecules in preparation of lipid nanoparticles, comprising: obtaining LNPs using the ionizable lipid molecule 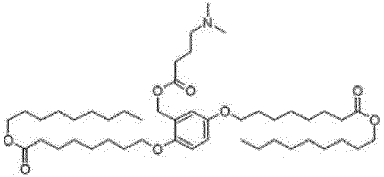 as well as phospholipid, cholesterol and polyethylene glycol through microfluidic synthesis method. The LNP can improve the translational expression level of its cargo, i.e. mRNA, in cells. Preferably, the phospholipid is DSPC (1,2-distearoyl-sn-glycerophosphatidylcholine) or DOPE (1,2-dioleoyl-sn-glycero-3-phosphoethanolamine). Further preferably, the polyethylene glycol is DMG-PEG2000. It should be noted that the microfluidic synthesis method is only a preferred method, and it is also possible to synthesize the LNP by thin film hydration followed by extrusion. These methods are not exhaustive, and all the methods for obtaining LNPs by synthesis using the XH-04 of the invention are within the protection scope of the invention.

Based on molar percentage, the formula for synthesizing the novel carrier LNP is as follows: XH-04: cholesterol: phospholipid: polyethylene glycol = 20-70%: 20-60%: 2-20%: 0.1-5%. Preferably, based on molar percentage, the formula for synthesizing the novel carrier LNP is as follows: XH-04: cholesterol: DSPC: DMG-PEG2000 = 31.5: 56: 10: 2.5 (mol%). It should be noted that the optimal formula has been verified in the following experiments, and those skilled in the art will appreciate that this range of molar percentages for formulas is a common flexible range and all the formulas can provide LNPs. All the LNPs obtained by such formulas are within the protection scope of the invention, and the experimental procedures and data are not further elaborated here. It should be noted that the combination of XH-04, cholesterol, DSPC and DMG-PEG2000 is not the sole one, but DSPC can be replaced with DOPE, and DMG-PEG2000 can be replaced with any kind of PEG (polyethylene glycol).

The specific steps of performance testing are as follows:
(1) Purification of LNPs by tangential flow filtration (TFF);
   It should be noted that in addition to tangential flow filtration, LNPs may be purified by dialysis plus concentration, these methods are not exhaustive, and all the methods that can purify the LNP obtained using the formula of the invention are within the protection scope of the invention.
(2) Verification of the performance of the LNP delivery carrier *in vitro* using spike protein_mRNA as the labelled mRNA.

The technical effects of the invention are verified by experiments as follows:
LNPs were prepared firstly through the following specific process:

### 1. Preparation of working fluid

(1) Mother liquor of each component (cholesterol: 10 mg/mL; 1,2-distearoyl-SN-glycero-3-phosphocholine (DSPC): 10 mg/mL; 1,2-dimyristoyl - RAC - glycero-3-methoxypolyethylene glycol 2000 (DMG-PEG2000): 4 mg/mL), and ionizable lipid molecule XH-04 (compound I, 10 mg) were formulated.
(2) 10 mg of XH-04 was fully dissolved in a total of 1 mL of anhydrous ethanol. XH - 04, 1,2-dimyristoyl - RAC - glycero-3-methoxypolyethylene glycol 2000 (DMG-PEG2000) and cholesterol were placed in a water bath of the water bath kettle at 37°C for 40 min; and 1,2-distearoyl-sn-glycero-3-phosphocholine (DSPC) was placed in a water bath of the water bath kettle at 55°C for 40 min.
(3) The preheated mother liquors of the components were added and mixed thoroughly according to the volumes as shown in Table 2 below, and then the mixture liquid was diluted by addition of 7.32 mL of anhydrous ethanol. The mixture was maintained in a water bath at 37°C.

**Table 2 [0144]**

| Component | XH-04 (or Dlin-MC3-DMA) | Cholesterol | 11,2-distearoyl-sn-glycero-3-phosphocholine (DSPC) | 1,2-dimyristoyl-RAC-glycero-3-methoxypolyethylene glycol 2000 (DMG-PEG2000) |
|---|---|---|---|---|
| Volume/uL | 1000 | 869 | 318 | 631 |

### 2. Synthesis of LNPs by microfluidics

LNPs were prepared using a NanoAssemblr microfluidic instrument with microfluidic chips provided by the manufacturer. After the microfluidic chip channels were cleaned and pretreated, the left syringe was filled with 8 mL of ultra-pure water, and the right syringe was filled with 2 mL of the mixture liquid of the lipid components. The parameters in the instrument control software were set as follows: total liquid flow rate: 16 mL/min, left and right flow rate ratio: 4: 1, initial discarded volume: 0.550 mL, and final discarded volume: 0.150 mL. This procedure was repeated several times to use up all the mixture liquid of the lipid components. All of the product LNP solutions were collected, and diluted by addition of ultra pure water within 30 min to have less than 0.5% of ethanol proportion in the solution.

### 3. Purification of LNPs by TFF ultrafiltration

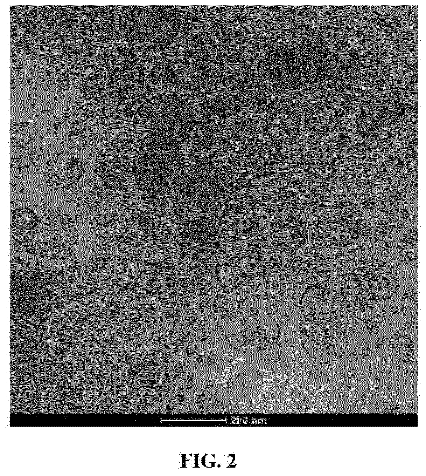

Liquid exchange was performed using a hollow fiber ultrafiltration membrane column to remove the ethanol component from the LNP solution, and the LNPs were dispersed in ultra-pure water and stored at 2-8°C. The cryo-EM image of the resulting XH-04LNP is as shown in Fig. 2.

LNPs with packaged spike protein_mRNA were prepared and compared with the existing LNP products in the experiment. The specific process is as follows:
Dlin-MC3-DMA ionizable lipid molecules (CAS No. 1224606-06-7) are a major component of existing LNP products. In this Example, MC3-LNP product was prepared and synthesized using Dlin-MC3-DMA and other essential components and used to package spike protein_mRNA as a control. At the same time, the ionizable lipid molecule XH-04 provided by the invention was used for replacing the Dlin-MC3-DMA molecule, which, together with the components like cholesterol, 1,2-distearoyl-SN-glycero-3-phosphocholine (DSPC), and 1,2-dimyristoyl - RAC - glycero-3-methoxypolyethylene glycol 2000 (DMG-PEG2000), was used to synthesize the XH04 -LNP product to package spike protein_mRNA. The mRNA delivery performance of XH04 LNP was verified *in vitro.*

### 1. Preparation of working fluid

(1) Mother liquor of each component (cholesterol: 10 mg/mL; 1,2-distearoyl-SN-glycero-3-phosphocholine (DSPC): 10 mg/mL; 1,2-dimyristoyl - RAC - glycero-3-methoxypolyethylene glycol 2000 (DMG-PEG2000): 4 mg/mL), and ionizable lipid molecule XH-04 or Dlin-MC3-DMA (10 mg) were formulated accurately.
(2) 10 mg of XH-04 (or Dlin-MC3-DMA) was fully dissolved in a total of 1 mL of anhydrous ethanol. XH - 04, 1,2-dimyristoyl - RAC - glycero-3-methoxypolyethylene glycol 2000 (DMG-PEG2000) and cholesterol were placed in a water bath of the water bath kettle at 37°C for 40 min; and 1 ,2-distearoyl-SN-glycero-3-phosphocholine (DSPC) was placed in a water bath of the water bath kettle at 55°C for 40 min.
(3) The preheated mother liquors of the components were added and mixed thoroughly according to the volumes as shown in the table below, and then the mixture liquid was diluted by addition of 7.32 mL of anhydrous ethanol. The mixture was maintained in a water bath at 37°C.

| Component | XH-04 (or Dlin-MC3-DMA) | Cholesterol | 1,2-distearoyl-SN-glycero-3-phosphocholinc (DSPC) | 1,2-dimyristoyl-RAC-glycero-3-methoxypolyethylene glycol 2000 (DMG-PEG2000) |
|---|---|---|---|---|
| Volume/uL | 1000 | 869 | 318 | 631 |

(4) 507 µg of spike protein_mRNA was taken and diluted to 39.37 mL with citric acid buffer (pH 6.0) and placed in a 50 mL centrifuge tube (on ice).

### 2. Synthesis of LNPs by microfluidics

LNPs were prepared using a NanoAssemblr microfluidic synthesizer with microfluidic chips provided by the manufacturer. After the microfluidic chip channels were cleaned and pretreated, the left syringe was filled with 8 mL of mRNA diluent, and the right syringe was filled with 2 mL of the mixture of the lipid components. The parameters in the instrument control software were set as follows: total liquid flow rate: 16 mL/min, left and right flow rate ratio: 4: 1, initial discarded volume: 0.550 mL, and final discarded volume: 0.150 mL. This procedure was repeated several times to use up all the mixture liquid of the lipid components. All of the product LNP solutions were collected, and diluted by addition of citric acid buffer within 30 min to have less than 0.5% of ethanol proportion in the solution.

### 3. Purification of LNPs by TFF ultrafiltration

Liquid exchange was performed using a hollow fiber ultrafiltration membrane column to remove components such as ethanol and citric acid from the LNP solution, and the LNPs were dispersed in the buffer for the preparation. The concentration of mRNA in the solution was measured and adjusted to 10 µg/mL, and the LNPs with packaged mRNA were sub-packaged and stored at -80°C.

### 4. In vitro verification of the performance of the LNP delivery carrier

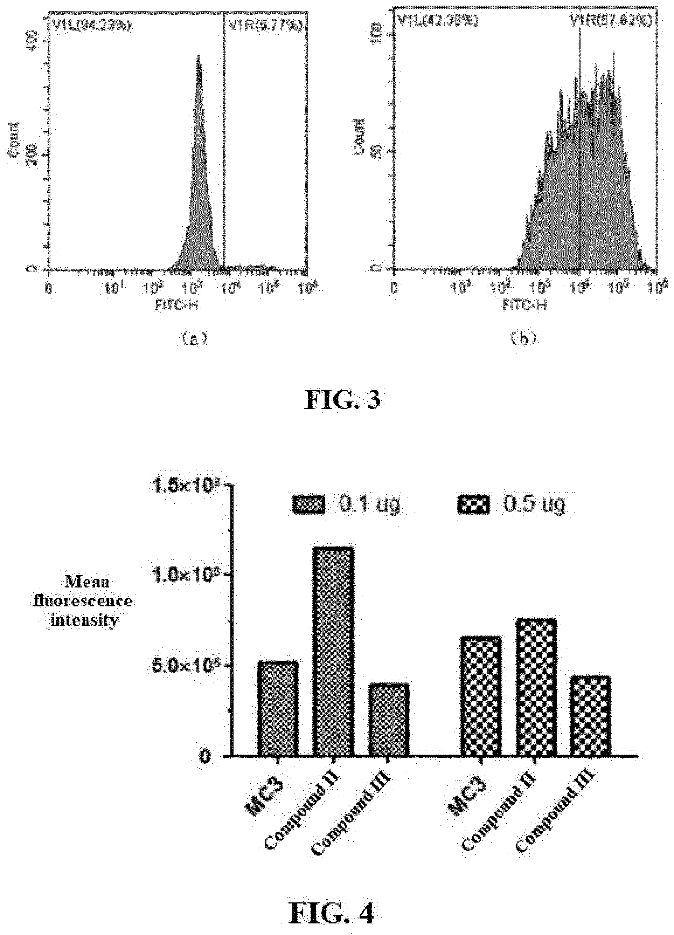

The LNP preparation with packaged mRNA was co-cultured with BHK cells for 72 h and the expression level of spike protein in the cells was detected by flow cytometry.
(1) Cell resuscitation and passage: The BHK-21 cells were resuscitated and cultured in culture flasks for passage to the desired number of cells.
(2) Plating: The cells in the culture flasks were digested and counted, plated in 96-well plates with 10,000 cells per well, and incubated overnight until the cells adhered.
(3) Dosing: The medium in the 96-well plate was blotted dry, washed 3 times by addition of PBS, and the liquid in the medium was blotted dry. To each well, 200 µL of the LNP preparation and 1800 µL of medium were added and cultured for 72 h in an incubator.
(4) The cells were collected and then incubated with the corresponding primary antibodies and fluorescent secondary antibodies for labeling, and the rate of positive cells was compared by flow cytometry, as shown in Fig. 3. It can be seen that the intracellular expression efficiency (57.62%) of mRNA supported by XH04-LNP is much higher than the efficiency (5.77%) of the existing product MC3-LNP.

The invention provides an ionizable lipid molecule XH-04 (compound I) with a novel structure, which, through preparation with lipid raw materials, obtains LNPs that has more excellent mRNA carrier performance and can obviously improve the translational expression level of its cargo, i.e. mRNA, in cells.

### Example 6: Preparation of lipid nanoparticle composition comprising enhanced green fluorescent protein mRNA

The ionizable lipid molecule compound II or compound III (10 mg), cholesterol, DSPC, and DMG - PEG2000 were dissolved in ethanol at a molar ratio of 38.5:50:10:1.5. Lipid nanoparticles (LNPs) were prepared with a weight ratio of total lipid to mRNA of 10:1. The mRNA was diluted to 0.2 mg/mL in 50 mM citrate buffer (pH 6). Using a syringe pump, the ethanol solution of the lipids was mixed with the aqueous mRNA solution at a ratio of 1:4 (volume/volume) at a total flow rate of 15 mL/min. The ethanol was then removed and the external buffer was replaced by PBS by dialysis. Finally, the lipid nanoparticles were filtered through a 0.22 µm pore sterile filter. The particle size of the lipid nanoparticles determined by quasi-elastic light scattering was 80-100 nm in diameter.

### Example 7: Evaluation of loading capacity of lipid nanoparticle composition

BHK cells were from ATCC and maintained at 37°C under 5% CO₂ in Dulbecco's modified Eagle medium (HyClone) supplemented with 10% fetal bovine serum (HyClone). Using lipid nanoparticles comprising enhanced green fluorescent protein mRNA, 0.1 µg or 0.5 µg of the lipid nanoparticle composition prepared in Example 6 or the commercial MC3 nanoparticle composition (Dlin - MC3 - DMA ionizable lipid molecule, CAS No. 1224606-06-7) was co-incubated with the cells to deliver therapeutic nucleic acids into BHK cells. After 24-hour incubation, the expression of intracellular green fluorescent protein was observed using a fluorescence microscope and the mean fluorescence intensity was calculated. The results are as shown in Fig. 4.

Referring to Fig. 4, it can be seen that after cell transfection with 0.1 µg or 0.5 µg of the lipid nanoparticle composition prepared by the compound II of the invention, the expression intensity of the intracellular green fluorescent protein exceeds that with the commercial MC3 nanoparticle composition; after cell transfection with 0.1 µg or 0.5 µg of the lipid nanoparticle composition prepared by the compound III of the invention, the expression intensity of the intracellular green fluorescent protein is close to that with the commercial MC3 nanoparticle composition, which indicates that the ionizable lipid molecule of the invention can effectively deliver therapeutic nucleic acids into BHK cells.

### Example 8: Evaluation of protein in vitro expression ability using lipid nanoparticle compositions comprising mRNA nucleic acid therapeutic agent

BHK cells were from ATCC and maintained at 37°C under 5% CO₂ in Dulbecco's modified Eagle medium (HyClone) supplemented with 10% fetal bovine serum (HyClone). 0.1 µg or 0.5 µg of the lipid nanoparticle composition prepared by the ionizable lipid compound of the invention or the commercial MC3 nanoparticle composition was co-incubated with the BHK cells to deliver the mRNA nucleic acid encoding the enhanced green fluorescent protein contained therein into the BHK cells to express the corresponding protein. After 24-hour co-incubation, the cells were digested with trypsin and collected, and signal intensity of intracellular green fluorescent protein was collected using a flow cytometer. Analysis of relative protein expression rates was performed using a cell group to which the commercial MC3 nanoparticle composition not containing mRNA encoding enhanced green fluorescent protein was added as a control. The results are as shown in Table 3.

**Table 3: Fluorescent protein expression rates in BHK cells for compositions of enhanced green fluorescent protein mRNA and the commercial lipid nanoparticles or the lipid nanoparticles prepared by the ionizable lipid of the invention at different concentrations**

| Ionizable lipid molecular No. | Fluorescent protein expression rate (0.1 µg) | Fluorescent protein expression rate (0.5 µg) |
|---|---|---|
| MC3 | 95.87% | 99.99% |
| I | 82.60% | 93.51 % |
| II | 99.53% | 99.98% |
| III | 88.41% | 99.89% |

The fluorescence intensity obtained after the commercial MC3 nanoparticle composition not containing mRNA encoding enhanced green fluorescent protein was co-incubated with cells is set as the base point, with the green fluorescent protein expression rate being 0. As shown in Table 3, green fluorescent protein expression effect equivalent to that of the commercial MC3 nanoparticle composition can be obtained using 0.5 µg of the lipid nanoparticle composition of the invention, indicating that the ionizable lipid molecule of the invention can effectively deliver therapeutic nucleic acids into BHK cells and has an excellent fluorescent protein expression rate.

The foregoing shows and describes the basic principles, main features and advantages of the invention. It should be understood by those skilled in the art that the above embodiments do not limit the invention in any way, and that all the technical solutions obtained by means of equivalent substitutions or equivalent transformations are within the scope of the invention.

## Claims

1. An ionizable lipid molecule, a pharmaceutically acceptable salt thereof and a stereoisomer thereof, the ionizable lipid molecule having a structure of formula (I): 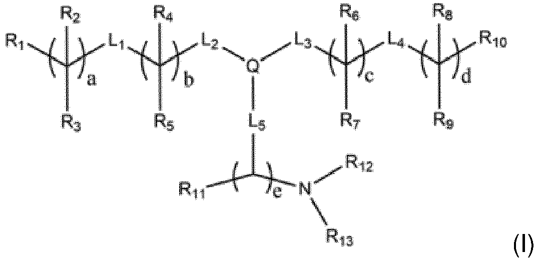 wherein,
Q is selected from a benzene ring, cyclopentyl, cyclohexyl, a pyrrole ring or a pyrimidine ring;
L₁ and L₄ are each independently selected from -O(C=O)-, -(C=O)O- or a carbon-carbon double bond;
L₂ and L₃ are each independently selected from -(CH₂)ₓO- or -O(CH₂)ₓ-, wherein x is an integer from 0 to 4;
L₅ is selected from -(CH₂)_{y}-O(C-O)-, -(CH₂)_{y}-(C=O)O- or -(CH₂)_{y}-O-, wherein y is an integer from 0 to 4;
L₂, L₃ and L₅ are each independently connected to any three sites in Q, wherein the sites are carbon or nitrogen;
a and d are each independently an integer from 0 to 18;
b and c are each independently an integer from 1 to 18;
e is an integer from 1 to 5;
R₁ and R₁₀ are each independently selected from methyl or cyclohydrocarbyl;
R₂ and R₃ are each independently selected from H or C₁-C₁₂ hydrocarbyl; or R₂ is selected from H or C₁-C₁₂ hydrocarbyl, and two carbon atoms connected to two adjacent R₃ form a carbon-carbon double bond;
R₄ and R₅ are each independently selected from H or C₁-C₁₂ hydrocarbyl; or R₄ is selected from H or C₁-C₁₂ hydrocarbyl, and two carbon atoms connected to two adjacent R₅ form a carbon-carbon double bond;
R₆ and R₇ are each independently selected from H or C₁-C₁₂ hydrocarbyl; or R₆ is selected from H or C₁-C₁₂ hydrocarbyl, and two carbon atoms connected to two adjacent R₇ form a carbon-carbon double bond;
R₈ and R₉ are each independently selected from H or C₁-C₁₂ hydrocarbyl; or R₈ is selected from H or C₁-C₁₂ hydrocarbyl, and two carbon atoms connected to two adjacent R₉ form a carbon-carbon double bond;
R₁₁ is independently selected from H or C₁-C₆ hydrocarbyl; and
R₁₂ and R₁₃ are each independently C₁-C₆ hydrocarbyl.

2. The ionizable lipid molecule, pharmaceutically acceptable salt thereof and stereoisomer thereof according to claim 1, wherein Q is selected from a benzene ring, cyclopentyl, or cyclohexyl; and preferably, Q is a benzene ring.

3. The ionizable lipid molecule, pharmaceutically acceptable salt thereof and stereoisomer thereof according to claim 1 or 2, wherein L₂, L₃, and L₅ are connected to Q in a form of 1,2,5-trisubstitution, 1,3,5-trisubstitution, 1,2,4-trisubstitution, 1,4,5-trisubstitution or 1,3,6-trisubstitution.

4. The ionizable lipid molecule, pharmaceutically acceptable salt thereof and stereoisomer thereof according to claim 1 or 2, wherein y is 0, 1, or 2.

5. The ionizable lipid molecule, pharmaceutically acceptable salt thereof and stereoisomer thereof according to claim 1 or 2, wherein a and d are each independently an integer from 6 to 10.

6. The ionizable lipid molecule, pharmaceutically acceptable salt thereof and stereoisomer thereof according to claim 1 or 2, wherein b and c are each independently an integer from 1 to 10.

7. The ionizable lipid molecule, pharmaceutically acceptable salt thereof and stereoisomer thereof according to claim 1 or 2, wherein e is selected from 2 or 3.

8. The ionizable lipid molecule, pharmaceutically acceptable salt thereof and stereoisomer thereof according to claim 1 or 2, wherein,
the cyclohydrocarbyl in R₁ and R₁₀ is selected from cyclopropyl, cyclobutyl, cyclopentyl, cyclohexyl or cycloheptyl;
the C₁-C₁₂ hydrocarbyl in the definitions of R₂ to R₉ is selected from C₁-C₁₂ alkyl, C₁-C₁₂ alkenyl, or C₁-C₁₂ alkynyl; and preferably C₁-C₆ alkyl;
R₁₁ is selected from H, methyl, ethyl, propyl, butyl, pentyl or hexyl; and preferably is methyl; and
R₁₂ and R₁₃ are each independently selected from methyl, ethyl, propyl, butyl, pentyl or hexyl; and preferably are methyl.

9. The ionizable lipid molecule, pharmaceutically acceptable salt thereof and stereoisomer thereof according to claim 1 or 2, wherein the ionizable lipid molecule is selected from the group consisting of: 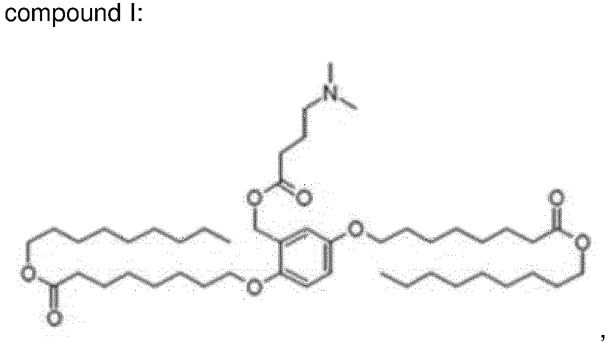 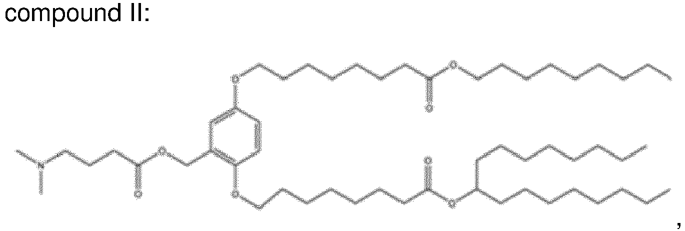 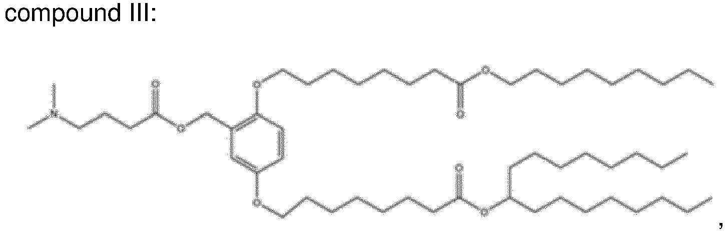 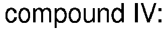 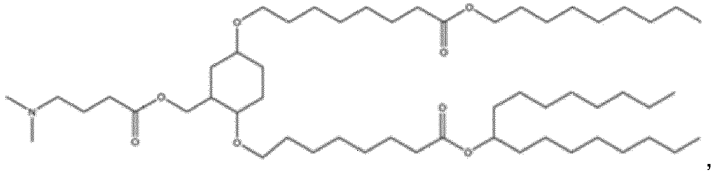 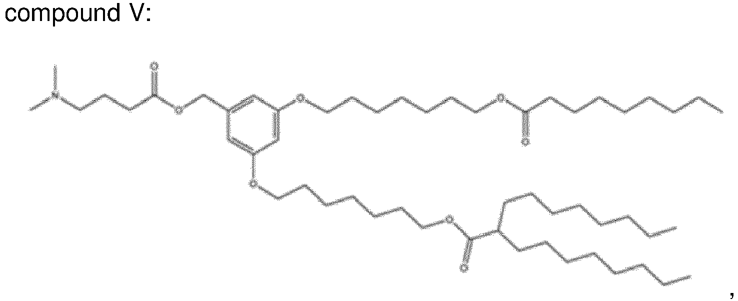 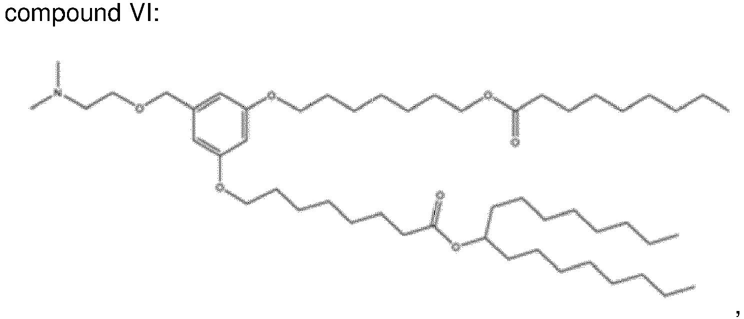 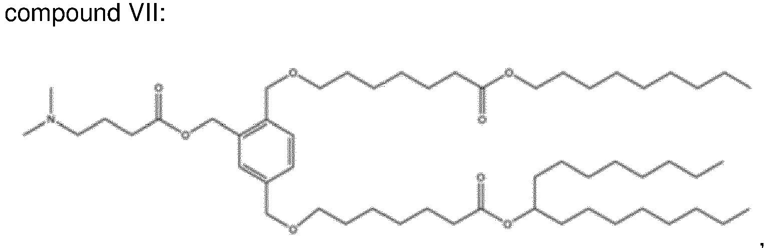 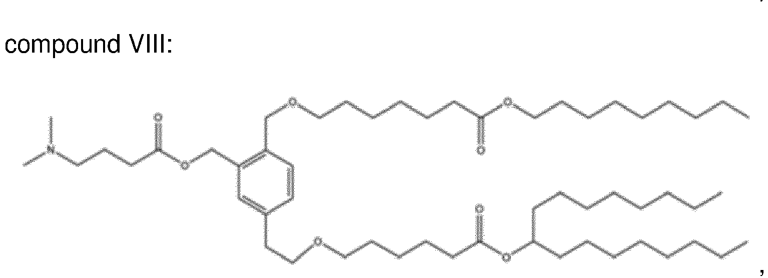 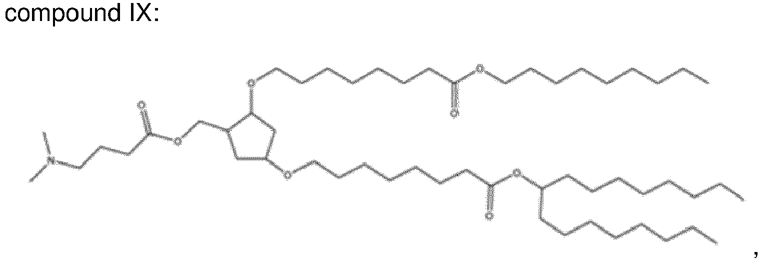 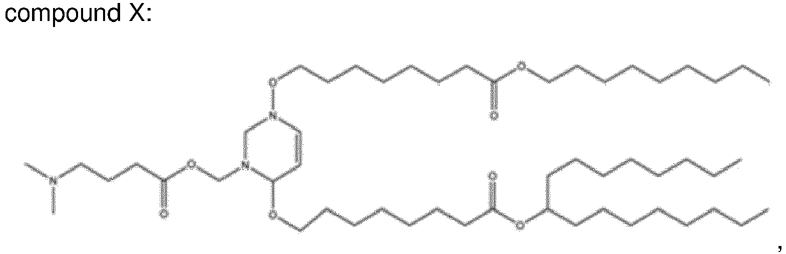 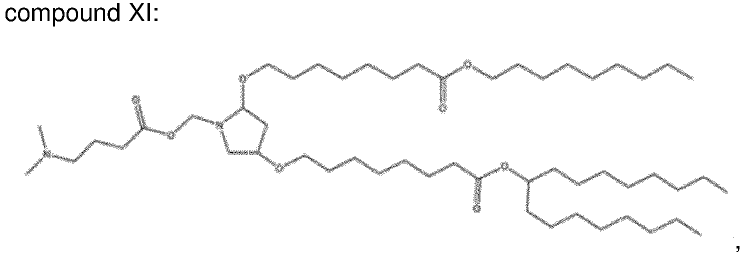 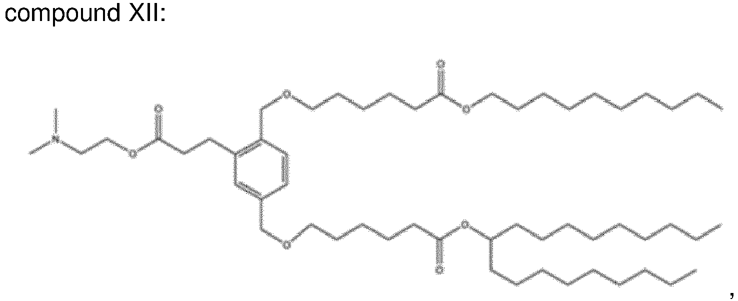 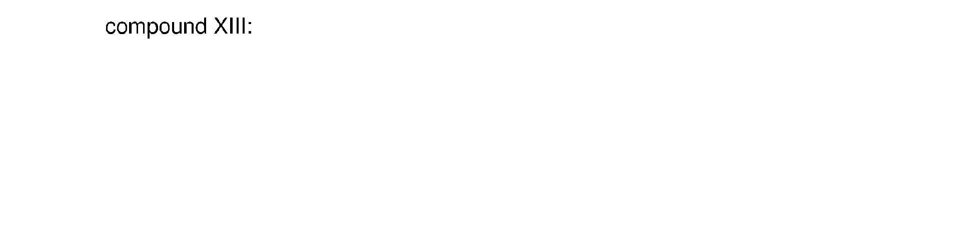 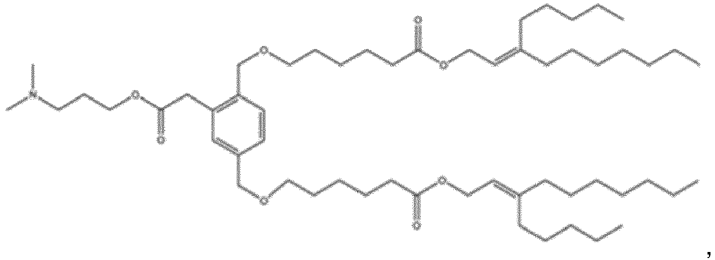 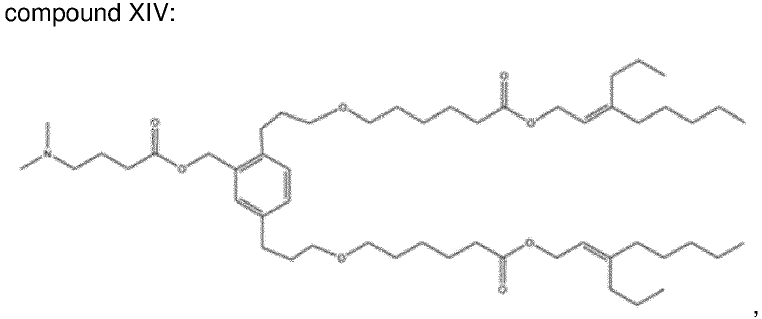 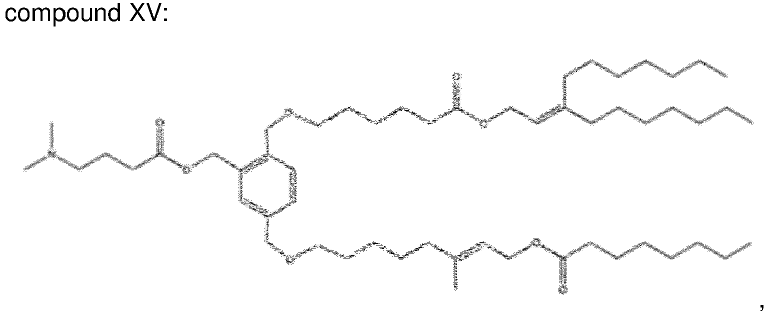 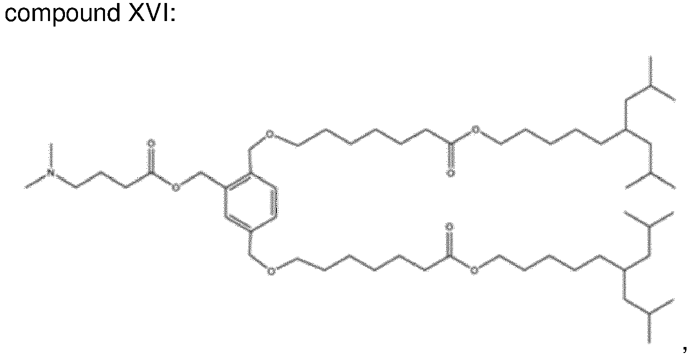 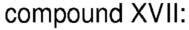 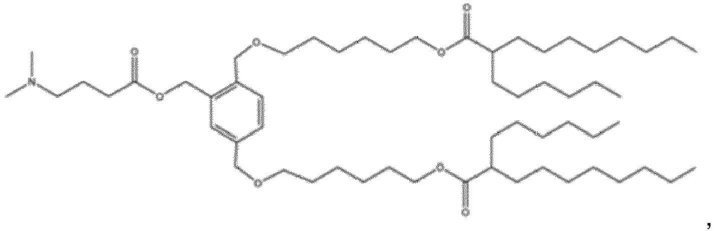 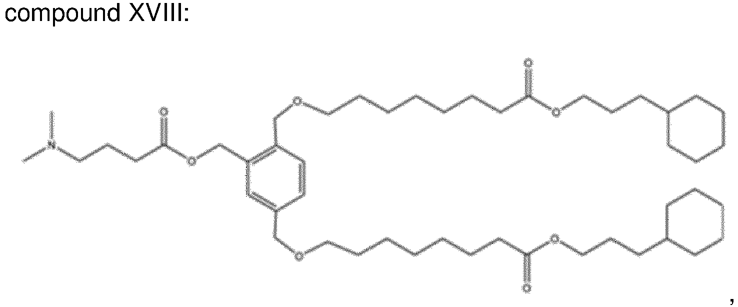 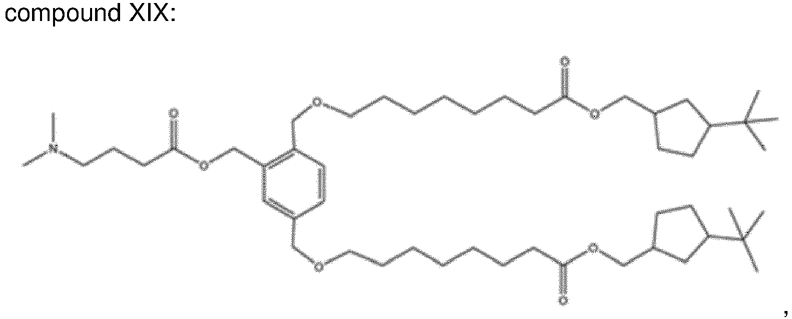 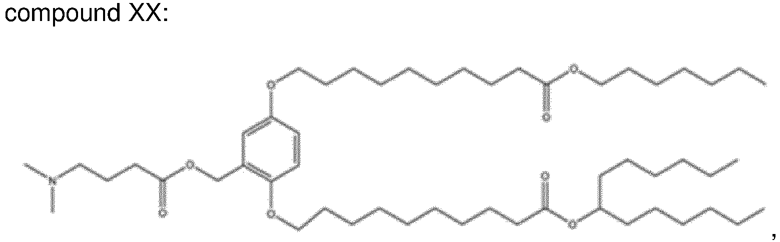 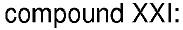 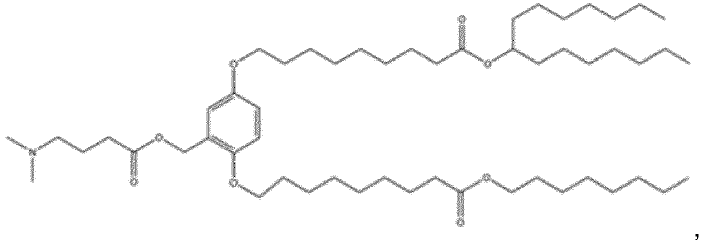 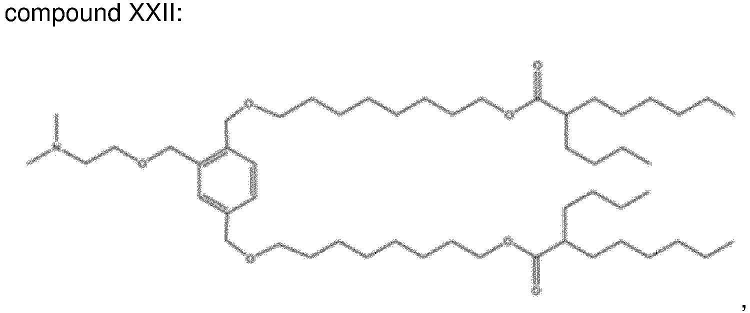 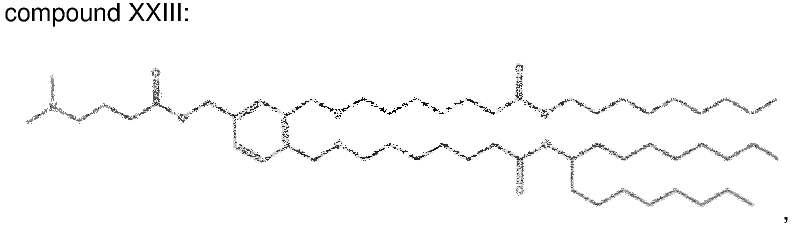 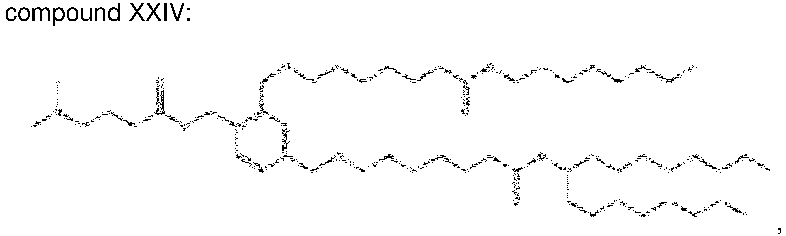 and

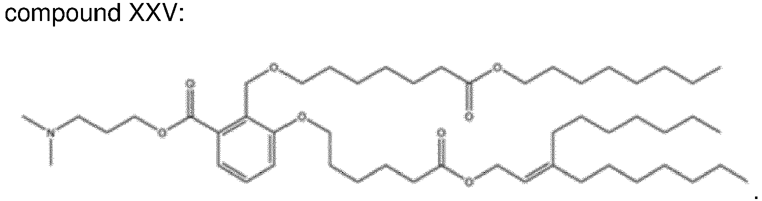

10. The ionizable lipid molecule, pharmaceutically acceptable salt thereof and stereoisomer thereof according to claim 1 or 2, wherein the ionizable lipid molecule is compound I:

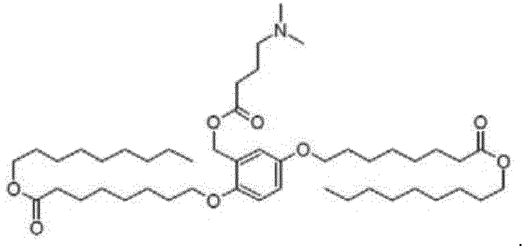

11. The ionizable lipid molecule, pharmaceutically acceptable salt thereof and stereoisomer thereof according to claim 1 or 2, wherein the ionizable lipid molecule is compound II:

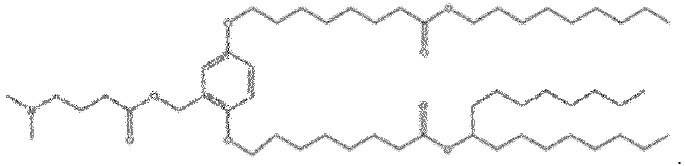

12. The ionizable lipid molecule, pharmaceutically acceptable salt thereof and stereoisomer thereof according to claim 1 or 2, wherein the ionizable lipid molecule is compound III:

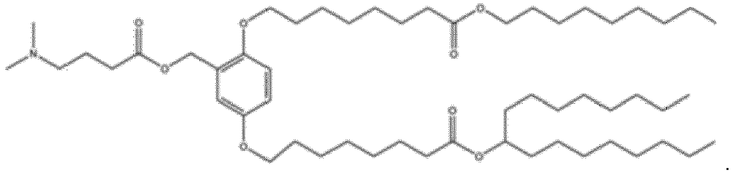

13. A preparation method of the ionizable lipid molecule, pharmaceutically acceptable salt thereof and stereoisomer thereof of any one of claims 1 to 10, wherein the ionizable lipid molecule is compound I, and the method comprises steps of:
step 1: to a mixed solution comprising 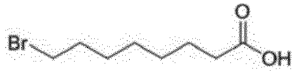 with dichloromethane (DCM) as a solvent, 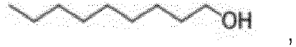 4-dimethylaminopyridine (DMAP) and triethylamine (TEA) are added; then 1-ethyl-(3-dimethylaminopropyl)carbodiimide hydrochloride (EDCI) is added to the solution with stirring for reaction; after completion of the reaction indicated by detection, the reaction solution is extracted, washed, dried, concentrated and purified to obtain 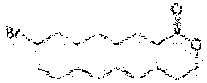 as a yellow oil;
step 2: to a mixed solution comprising 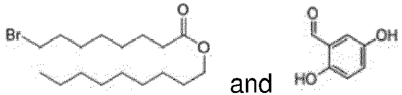 with N,N-dimethylformamide (DMF) as a solvent, Cs₂CO₃ is added, the mixture is stirred to react, and after completion of the reaction indicated by detection, the reaction solution is extracted, washed, dried, concentrated and purified to obtain 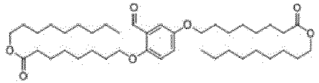 as a white solid;
step 3: to a mixed solution of tetrahydrofuran (THF), ethanol and 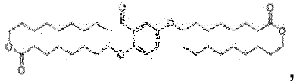 NaBH₄ below 0°C is added, the mixture is stirred to react, and after completion of the reaction indicated by detection, the reaction solution is extracted, washed, dried, and concentrated to obtain as a white solid; and 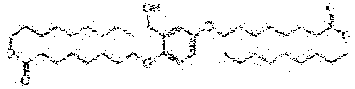
step 4: 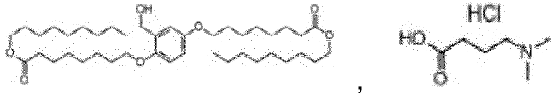 and 1-ethyl-(3-dimethylaminopropyl)carbodiimide hydrochloride (EDCI) are stirred to react in a pyridine solution, and after completion of the reaction indicated by detection, the reaction solution is extracted, washed, dried, concentrated and purified to obtain an ionizable lipid molecule 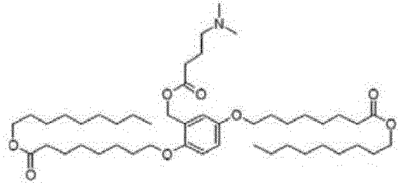 as a colorless oil.

14. The preparation method according to claim 13, wherein the method for detecting completion of the reaction is silica gel plate thin layer chromatography (TLC).

15. A composition comprising the ionizable lipid molecule, pharmaceutically acceptable salt thereof and stereoisomer thereof of any one of claims 1 to 12, and a therapeutic agent.

16. The composition according to claim 15, wherein the therapeutic agent includes a nucleic acid.

17. The composition according to claim 16, wherein the nucleic acid is selected from small interfering RNA (siRNA), microRNA (miRNA), and messenger RNA (mRNA).

18. The composition according to claim 17, wherein the mRNA is non-self-replicating mRNA or self-replicating mRNA.

19. The composition according to any one of claims 15 to 18, wherein the composition further comprises one or more excipients selected from a neutral lipid, a steroid, and a polymer-conjugated lipid;
preferably, the neutral lipid is selected from one or more of 1,2-distearoyl-sn-glycerophosphatidylcholine (DSPC), dipalmitoyl phosphatidylcholine (DPPC), dimyristoyl phosphatidylcholine (DMPC), dioleoyl phosphatidylcholine (DOPC), palmitoyl oleoyl phosphatidylcholine (POPC), and 1,2-dioleoyl-sn-glycero-3-phosphoethanolamine (DOPE); and more preferably DSPC and DOPE;
the steroid is selected from cholesterol, sitosterol, fucosterol, campesterol, and stigmasterol; and more preferably is cholesterol; and
the polymer-conjugated lipid is polyethylene glycol, preferably PEGylated DMG, and more preferably DMG-PEG2000.

20. The composition according to claim 19, wherein the molar ratio of the ionizable lipid molecule to the neutral lipid is 2:1 to 8:1, the molar ratio of the ionizable lipid molecule to the cholesterol is 2:1 to 1:1; and the molar ratio of the ionizable lipid molecule to the PEGylated DMG is 100:1 to 25:1.

21. Use of the ionizable lipid molecule, pharmaceutically acceptable salt thereof and stereoisomer thereof according to any one of claims 1 to 12 in preparation of a carrier for delivery of a nucleic acid to a cell.

22. The use according to claim 21, wherein the nucleic acid is selected from small interfering RNA (siRNA), microRNA (miRNA), and messenger RNA (mRNA).

23. The composition according to claim 22, wherein the mRNA is non-self-replicating mRNA or self-replicating mRNA.

24. Use of the ionizable lipid molecule, pharmaceutically acceptable salt thereof and stereoisomer thereof of any one of claims 1 to 12 in preparation of a lipid nanoparticle, comprising obtaining a liposome nanoparticle (LNP) by synthesis from the ionizable lipid molecule, as well as phospholipid, cholesterol, and polyethylene glycol, the LNP being capable of improving the translational expression level of its cargo, i.e. mRNA, in a cell.

25. The use according to claim 24, wherein the ionizable lipid molecule is selected from compounds I to III: 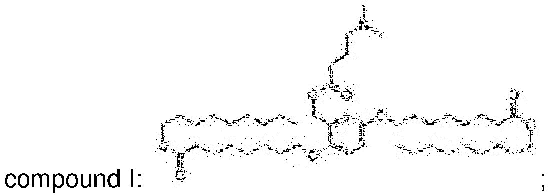 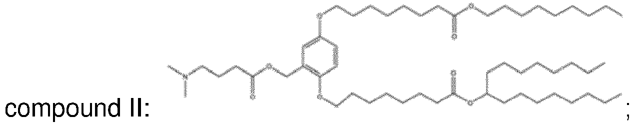 and

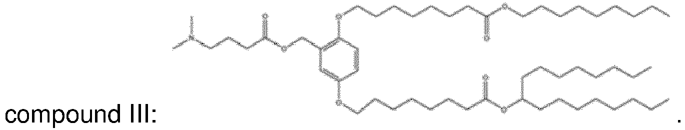

26. The use according to claim 24, 25 or 26, wherein the phospholipid is DSPC or DOPE; and the polyethylene glycol is DMG-PEG2000.

27. The use according to claim 24, 25 or 26, wherein the formula for synthesis of the carrier LNP is based on the following molar percentages: ionizable lipid molecule: cholesterol: phospholipid: polyethylene glycol = 20-70%: 20-60%: 2-20%: 0.1-5%.

28. The use according to claim 24, 25 or 26, wherein, when compound I is used, the formula for synthesis of the carrier LNP is based on the following molar percentages: ionizable lipid molecule: cholesterol: DSPC: DMG-PEG2000 = 31.5%: 56%: 10%: 2.5%; or,
when compound II or compound III is used, the formula for synthesis of the carrier LNP is based on the following molar percentages: the formula for synthesis of the carrier LNP is based on the following molar percentages: ionizable lipid molecule: cholesterol: DSPC: DMG-PEG2000 = 38.5%: 50%: 10%: 1.5%.

29. The use according to claim 24, 25 or 26, wherein the LNP is obtained by a microfluidic synthesis method using the ionizable lipid molecule, as well as phospholipid, cholesterol, and polyethylene glycol.

30. The use according to claim 29, wherein the microfluidic synthesis method comprises steps of:
preparing a mixed solution of the ionizable lipid molecule as well as phospholipid, cholesterol and polyethylene glycol;
preparing an mRNA diluent;
preparing the LNP using a microfluidic synthesizer with microfluidic chips provided by the manufacturer: the mRNA diluent is loaded into a syringe on one side, the mixed solution is loaded into a springe on the other side, parameters are set, the synthesis procedure is repeated several times to use up all the mixed solution, all the product LNP solutions are collected, and a citric acid buffer is added within 30 min to dilute the solution to have less than 0.5% of ethanol proportion in the solution.

31. The use according to claim 29 or 30, wherein the microfluidic synthesis method further comprises purification by tangential flow filtration to obtain the LNP.
